# EUROPÄISCHE PATENTSCHRIFT

(11) **EP 3 883 497 B1**
(45) Veröffentlichungstag und Bekanntmachung des Hinweises auf die Patenterteilung: **23.04.2025**
(21) Anmeldenummer: 19817579.6
(22) Anmeldetag: 19.11.2019
(51) Int. Cl.: A61F 2/16

(54) **VORRICHTUNG ZUR AUFNAHME EINER INTEROKULAREN LINSE UND VERFAHREN ZUM FALTEN EINER INTEROKULAREN LINSE**
DEVICE FOR RECEIVING AN INTRAOCULAR LENS, AND METHOD FOR FOLDING AN INTRAOCULAR LENS
DISPOSITIF DE LOGEMENT D'UNE LENTILLE INTRAOCULAIRE ET PROCÉDÉ DE PLIAGE D'UNE LENTILLE INTRAOCULAIRE

(30) Priorität: 19.11.2018 CH 14292018
(43) Veröffentlichungstag der Anmeldung: 29.09.2021
(73) Patentinhaber: Medicel AG, 9423 Altenrhein (CH)
(72) Erfinder: DOCKHORN, Volker, 9423 Altenrhein (CH)
(74) Vertreter: Swisspat Riederer Hasler Patentanwälte AG
(86) Internationale Anmeldenummer: PCT/CH2019/050026
(87) Internationale Veröffentlichungsnummer: WO 2020/102919

(56) Entgegenhaltungen:
- EP-A2- 2 306 932
- CH-A1- 709 039
- US-B2- 8 668 734

## Beschreibung

### TECHNISCHES GEBIET DER ERFINDUNG

Die vorliegende Erfindung betrifft eine Vorrichtung zur Aufnahme einer intraokularen Linse und ein Verfahren zum Falten einer intraokularen Linse.

### HINTERGRUND DER ERFINDUNG

Bei Kataraktoperationen werden heutzutage standardmässig künstliche Linsen, sogenannte Intraokularlinsen, in den Kapselsack des Auges eingesetzt.

Bei der Operation wird eine okulare Inzision von typischerweise 2 bis 4 mm gemacht, durch welche die natürliche Augenlinse zunächst entfernt und dann das Implantat eingesetzt wird. Zum Einsetzen wird die künstliche Linse in gefaltetem Zustand durch die Inzision in den Kapselsack eingeführt. Sobald die gefaltete Linse in den Kapselsack eingeführt ist, entfaltet sich diese wieder in ihre ursprüngliche Form,

Die heute üblichen künstlichen Linsen bestehen aus einem optischen Linsenkörper und in der Regel zwei oder mehreren von diesem quer zur optischen Achse des Linsenkörpers peripher abstehenden Haptiken, welche im Kapselsack als Positionsfedern für den Linsenkörper dienen. Beispielsweise stehen zwei Haptiken, welche am Linsenkörper einander gegenüberliegend angeordnet sind, gleichgesinnt spiralierend vom Linsenkörper ab. Diese weltweit am häufigsten verwendete Haptikform wird in der Fachbranche als open c-loop Haptik bezeichnet.

Verbesserte Operationswerkzeuge und Implantate ermöglichen es den Chirurgen, die Inzisionen zusehends kleiner zu machen. Die Entfernung der natürlichen Augenlinse kann heutzutage bereits durch Inzisionen von weniger als 2 mm erfolgen. Dies macht jedoch nur Sinn, wenn auch die intrackulare Linse durch eine derartig kleine Inzision eingesetzt werden kann.

Zum Einsetzen einer intraokolaren Linse sind in den vergangenen Jahren Linsenträger oder Kartuschen entwickelt worden, in welche eine Linse ladbar und sodann mittels eines Injektors aus dem Linsenträger ausgestossen werden kann.

Beispiele für solche Linsenträger oder Kartuschen und Injektoren sind zum Beispiel aus den Patentschriften US 6 267 768, US 5 810 833, US 6 283 975, US 6 248 111, US 4 681 102, US 5 582 614, US 5 499 987, US 5 947 975, US 6 355 046 und EP 1 290 990 B1, sowie den Offenbarungen US 2004/0199174 A1, EP 1 905 386 A1 und WO 03/045285 A1 bekannt

Bei der Injektorvorrichtung gemäss der US 4 681 102 sind die Kartusche, die als Faltvorrichtung für die Linse ausgebildet ist, und die Injektordüse separate Teile. Die Kartusche kann in das Injektorgehäuse eingeschoben werden, worauf die Injektordüse vom am Injektorgehäuse aufgeschraubt werden kann.

Bei der Injektorvorrichtung gemäss der US 5582614 und den meisten vorbekannten Injektorvorrichtungen, wie z.B. US 6 267 768, US 5 810 833, US 6 283 975 und US 6 248 111, besteht die Kartusche einstückig aus einer Faltvorrichtung und einer Injektordüse.

Intraokularlinsen werden vom Hersteller steril verpackt und gegebenenfalls in einem Flüssigkeitsbad geliefert. Je nach Linsenmaterial kann die Lagerung in einer Flüssigkeit notwendig sein, um die Linse vor Austrocknung zu schützen. Bei der Operation muss die Linse im sterilen Bereich der Verpackung entnommen und in die Ladevorrichtung eines Injektors oder mit mitgelieferter Kartusche direkt in einen Injektor eingesetzt bzw. geladen werden. Die Linsen sind sehr empfindliche Gebilde, welche beim Umladen in eine Kartusche, beim Falten oder beim Ausstossen aus der Injektordüse leicht beschädigt werden können. Die Beschädigungsgefahr ist insbesondere gross für die Haptik, welche den optischen Teil der Linse umgibt. Insbesondere beim Ausstossen der Linse aus dem Injektor besteht die Gefahr, dass eine der beiden Haptik eingeklemmt und demzufolge abgerissen wird oder die vordere Haptik dem Linsenkörper vorauseilend sich verfrüht im Auge ausbreitet, was problematisch sein kann, wie weiter unten gezeigt.

Eine übliche Kartuschenkonstruktion, wie die EP 1 290 990 B1, WO 03/045285 A1, EP 1 905 386 A1, US 5 582 614 und US 5 499 987 zeigen, weist zwei durch ein einziges Scharnier verbundene Halbschalen auf, sei es mit oder ohne Rillen oder Halteeinrichtung zum Erfassen der Linsenränder.

Die Offenbarung WO 03/045285 A1 zum Beispiel zeigt ein Verfahren zum Einführen einer intraokularen Linse in den Kapselsack des Auges, bei welchem ein Überdruck erzeugt wird, um eine in einem Gleitmittel schwimmend aufgenommene Linse aus der Injektordüse auszustossen. Ein kompressibler und deformierbarer Kolben passt sich dem sich nach vorn verengenden Düsenkanal kontinuierlich an. Die Linse wird auf ihrem Weg weiter gefaltet und hat am Ende ihres Weges einen sehr kleinen Durchmesser. Aufgrund der Deformierbarkeit des Kolbens, kann das Ende des Düsenkanals sehr eng gehalten sein, folglich ist lediglich eine sehr kleine Inzision nötig. Ein Set zur Ausführung des Verfahrens enthält einen Linsenträger und eine Linse. Die Linse befindet sich in spannungsfreiem Zustand im Linsenträger, Linse und Linsenträger sind vorzugsweise von einem Halter getragen und bis zur Verwendung in einer Packung steril verpackt, und zwar im Fall einer hydrophilen Linse in einer Flüssigkeit, welche die Linse vor dem Austrocknen schützt Bei der Operation wird der Linsenträger samt der darin gelagerten Linse der Packung entnommen, in den Injektor eingesetzt und gefaltet. Hierauf wird durch den Kanal eine Gleitflüssigkeit eingefüllt. Die Linse kann nun in den Käpselsack des zu behandelnden Auges injiziert werden.

Im Allgemeinen können im Stand der Technik Systeme für vorgefaltete Linsen und Systeme für nicht vorgefaltete Linsen unterschieden werden. Bei den Systemen ohne vorgefaltete Linsen, werden die Linsen erst während dem Stossprozess zur Injektion gefaltet Ein Beispiel eines derartigen Systems ohne vorgefaltete Linsen, ist in der Patentschrift US 8,668,734 B2 offenbart. Hier wird eine Kartusche aus zwei Formteilhälften verwendet, in welche die Linse bei geöffnetem Formteil eingelegt wird. Dieses System sieht zudem für die hintere Haptik eine Aussparung seitlich der Stossrichtung des Kolbens vor, während die vordere Haptik distal gelagert ist. Es werden jedoch insbesondere Systeme verwendet, bei welchen die Linse von Hinten, noch unverformt bzw. ungefaltet in eine Ladekammer geladen wird (wie dies z.B. in US 5,810,833 gezeigt wird). Die hintere Öffnung, in die die Linse eingeschoben wird, ist mindestens so breit und hoch, wie die Linse selbst. Sofern ein Kolben mit deformierbarer Spitze (in der Regel aus Silikon oder TPE) verwendet wird, dann muss diese Kolbenspitze das gesamte Volumen der hinteren Öffnung ausfallen, um nicht Gefahr zu laufen, mit dieser Kolbenspitze die hintere der beiden Haptiken zu überfahren und damit einzuklemmen. Dieses grosse Volumen der deformierbaren Kolbenspitze führt zu hohen Systemkräften, wenn der Kolben bis in den maximal verjüngten Bereich der Cartridgespitze vorgeschoben wird. Die hierdurch bewirkten Kräfte können sogar grösser sein, als die durch die Linse selbst bewirkten Kräfte. Zudem limitiert die Dehn- oder Komprimierbarkeit der voluminösen Kolbenspitze den minimal notwendigen Innendurchmesser der vorderen Düsenspitze. Aus diesem Grund werden bis heute immer noch häufig Systeme verwendet, bei denen die Linsen vor dem Stossprozess zur Injektion gefaltet bzw. vorgefaltet werden. Hierfür werden insbesondere Flügelkartuschen (wie z.B. in US 6,267,768, US 6,248,111, US 5,947,975 oder US 4,681,102 offenbart) verwendet, welche immer aus zumindest zwei zunächst geöffneten Halbschalen bestehen. Die Linse wird beim Schliessen der zumindest zwei Halbschalen vorgefaltet und liegt in der Ladekammer in vorgefaltetem Zustand vor, Das Vorfalten der Linse samt Haptiken durch Schliessen der Flügelkartusche reduziert das Innenvolumen der geschlossenen Flügelkartusche auf nahezu die Hälfte und ermöglicht so die Verwendung kleinerer deformierbarer Kolbenspitzen, welche bei gleich grossem Innendurchmesser der Cartridgespitze kleinere Inzisionskräfte bewirken respektive bei gleicher Inzisionskraft die Verwendung kleinerer Cartridge-Innendurchmesser und damit kleinerer Inzisionen bewirken. Der Nachteil dieser Systeme zum Vorfalten der Linsen ist, dass insbesondere die Linsenhaptik, im schlimmsten Fall sogar die Optik beim Schliessen der beiden Flügel der Kartusche zwischen den Flügeln eingeklemmt werden kann. Eingeklemmte Haptiken reissen in der Regel beim weiteren Vorschieben der Linse ab, was einem Totalschaden der Linse gleichkommt.

In den Offenbarungen WO 2015/070358 A2 und CH 709039 A1 werden Flügelkartuschen zur Aufnahme einer intraokularen Linse in einer Ladekammer vorgestellt. Die Flügelkartusche wird von einer ersten und einer zweiten Halbschale gebildet, wobei jede Halbschale längsseitig einen Flügelgriff aufweist. Die beiden Halbschalen sind an der jeweils flügellosen Längsseite über ein erstes Gelenk gelenkig miteinander verbunden und können mittels des ersten Gelenks relativ zueinander von einer offenen Stellung in eine geschlossene Stellung bewegt werden, wobei die beiden Halbschalen in geschlossener Stellung einen Ausstosskanal für eine interokulare Linse bilden. Weiter ist in der Offenbarungsschrift beschrieben, dass eine vordere c-loop Haptik vorgefaltet wird, indem die vordere Haptik, welche düsenseitig von einem Stopper blockiert wird, durch Vorschub des Stempels und indirekt über den Vorschub der Linse an die Optik gedrückt wird. Hierdurch wird verhindert, dass die vordere Haptik in gestreckter Form ins Auge injiziert wird. Anfangs macht die Haptik dabei einen Bogen, je länger die Haptik ist, desto mehr wird dieser Bogen beim weiteren Vorschub in der Düse zusammengedrückt. Je nach Haptikdesign kann es dazu führen, dass die Haptik quasi in der Mitte ihrer Länge "gefaltet" wird und damit die gefaltete, aber immer noch der Länge nach gestreckte Haptik ins Auge eintritt. Durch diese Faltung kann die Haptik dann immer noch die halbe Länge haben, was von den operierenden Ärzten mit Blick auf eine mögliche Kapselsackbeschädigung als störend empfunden. werden kann.

Bei hydrophilen Linsen besteht zudem das Problem, dass die vordere Haptik, wenn sie in einer Flügelkartusche nur düsenseitig an die Optik gedrückt wird, sehr schnell im Auge entfaltet, noch bevor die Optik selbst ins Auge kommt. Damit ergibt sich der ungünstige Fall einer im Prinzip gestreckt ins Auge kommenden Haptik Der der Haptik nachfolgende Linsenkörper folgt der Bewegung der Haptik und kann dadurch bei Eintritt ins Auge ggf. um 180° drehen (Oberseite nach unten und Unterseite nach oben). Dies ist sehr störend für den Chirurgen, da er zur Korrektur die Linse im Auge bei engen Platzverhältnissen drehen muss.

In den beiden vorgenannten Situationen wäre es wünschenswert, dass die vordere Haptik nicht nur düsenseitig an die Optik gedrückt wird, sondern vielmehr derart auf der Optik zu liegen kommt, dass die Haptik beim Falten bzw. weiteren Falten der Optik von deren Rändern gefasst bzw. eingeschlossen wird und erst entfalten kann, wenn sich nach Injektion die Optik im Auge entfaltet und dadurch die vordere Haptik freigibt. Ein solChes Falten wird auch als Sandwichfaltung bezeichnet. Es ist die typischste Form der Haptikfaltung bei allen Kartuschen, die ohne Vorfaltung (und deshalb ohne Flügel) funktionieren und die Faltung erst und ausschliesslich bei Vorschub durch die Innengeometrie der Kartusche erfolgt. WO 2014/74860 A1 und EP 2'916'769 B1 beschreiben eine typische Ausführungsform einer solchen Kartusche ohne Vorfaltung. Die vordere Haptik wird mittels des kleinen Schlitzes am hintersten Ende der Kartusche seitens des Bedieners (OP-Schwester oder Chirurg) bereits beim Einlegen der Linse in die Kartusche auf die Optik gelegt. Diese Form des Ladens einer Linse durch den Anwender wird unabhängig vom Injektorsystem als ein nicht-vorgeladener Injektor bezeichnet. Als vorgeladene Linsen werden hingegen jene Linsen bezeichnet, die bereits beim Linsenhersteller in die Ladekammer des Injektors eingelegt wurden und mit diesem zusammen sterilisiert wurden. Eine Kartusche entsprechend WO 2014/074860 A1 kann nur für nicht-vorgeladene Linsen verwendet werden, da die Linse samt Haptik in entspannter Position und über die gesamte Lebensdauer des Produktes hinweg bereits in der Ladekammer gelagert sein muss, während WO 2014/074860 das aktive Einfädeln der vorderen Haptik durch den Schlitz am Kartuschenende durch den Endanwender (OP-Schwester oder Chirurg) voraussetzt. Ähnlich dazu zeigt auch die Offenlegungsschrift US 2009/0270876 eine geschlossene, von hinten zu beladende Kartusche. Auch hier ist ein aktives Bedienen und Beladen durch den Endanwender nötig. Ein entspanntes Vorladen ist auch hier nicht möglich.

Bei einem vorgefalteten System (d.h. bei einer Flügelkartusche) hingegen ist es bis anhin sowohl für vorgeladene als auch nicht-vorgeladene Linsen äusserst schwierig die Haptik derart auf die Optik zu legen, dass Sandwichfaltung erreicht wird, d.h. die Haptik derart geklemmt wird, dass diese sich erst im Auge von der Optik lösen kann, da beim Schritt des Vorfaltens sowohl Optik als auch Haptik in eine U-Form gebracht werden und die vordere Haptik damit auch beim weiteren Vorschub der Linse nicht kontrolliert und reproduzierbar auf die Oberseite der Linsenoptik gelangt, sondern meistens weiterhin nur gegen die Optik gedrückt wird. Zudem erhöht sich bei Flügelkartuschen das Risiko massiv, dass die vordere Haptik beim Schliessen der Ladekammer zwischen den Flügeln einklemmt, wenn es zuvor gelungen ist, die Haptik auf die Optik zu legen.

In der Offenlegungsschrift 3562/CHE/2014 (indische Patentanmeldung in Prüfung mit dem Titel "Leading Haptic Positioner for Preloaded IOL Delivery System" von R.D. Thularsiraj) wird ein Verfahren zur Sandwichfaltung ausgehend von einem vorgeladenen System vorgestellt. Diese Sandwichfaltung für ein vorgeladenes System funktioniert derart, dass mittels eines manuell verschiebbaren Hakens oder auch Schiebers, der durch die Düsenspitze in die Ladekammer eingeführt wird, die Haptik manuell auf die Optik gelegt wird. Sobald die Flügel der Ladekammer geschlossen wurden, wird der Haken wieder herausgezogen.

Nachteile dieser Methode bestehen darin, dass mindestens zwei zusätzliche Bauteile benötigt werden, die das System teurer machen und für den Arzt entstehen durch das Vorschieben des Schiebers und das spätere Herausziehen des Schiebers zwangsläufig zwei zusätzliche Arbeitsschritte. Da vorgeladene Systeme häufig anhand der Anzahl der Vorbereitungsschritte mit einander verglichen werden, stellt dieses zusammen mit den zusätzlichen Kosten einen Wettbewerbsnachteil dar.

Ein anderer Nachteil dieses Konzeptes besteht darin, dass dieser Ansatz nur für voll-vorgeladene Linsen (in der Regel für hydrophobe Linsen verwendet) verwendet werden. Er ist jedoch nicht nutzbar für sogenannte halb-vorgeladene Linsen (in der Regel für hydrophile Linsen verwendet). Bei letzteren ist die Linse zwar in der Ladekammer vorgeladen, mindesten aber die Ladekammer separat vom restlichen Injektor in Flüssigkeit gelagert. Da der Schieber in der Düse sitzt, kann der Haken am Ende des Schiebers nicht in Kontakt mit der separat gelagerten Linse in der Ladekammer positioniert sein. Wenn die Ladekammer nach Entnahme aus der Lagerungsflüssigkeit in den Injektor eingesetzt wird, dann muss der Haken durch den Schieber soweit zurückgezogen sein, dass er das Einsetzen der Ladekammer in den Injektor nicht behindert. Aufgrund der geringen Grössenverhältnisse ist es schwierig zu bewerkstelligen, dass ein weit zurückgezogener Haken, die Haptik präzise greift und positioniert. Bei voll-vorgeladenen Linsen besteht diese Problematik nicht, da die Haptik in der Regel schon werkseitig an den Haken angelegt (positioniert) wird.

Ein weiterer Nachteil dieses Konzeptes besteht darin, dass der Haken durch die Düse eingeführt werden muss, die maximale Grösse des Hakens und Schiebers besteht daher in der Grösse der Düse an deren Spitze. Bei sehr kleinen Inzisionen, kann der Innendurchmesser einer Spitze zwischen 1 mm und 1.5 mm liegen, was zu sehr filigrauen Haken- und Schiebergeometrien führt. Je filigraner das System ausgebildet ist, desto schwieriger ist es, ein zuverlässiges System zu erstellen, dass die Haptik sicher erfasst und positioniert.

### AUFGABE

Es ist daher Aufgabe der vorliegenden Erfindung, eine alternative Vorrichtung oder ein alternatives System für vorgeladene intraokulare Linsen zu schaffen, bei welchem die vordere Haptik beim Ausstossen aus der Injektordüse bzw. beim Injizieren in ein Auge nicht absteht oder vorsteht. Insbesondere wäre es wünschenswert die vordere Haptik von Linsen, insbesondere von c-loop Linsen, zwecks Injizierens in ein Auge sicher und reproduzierbar auf die Optik zulegen, insbesondere derart, dass die vordere Haptik beim Injizieren nicht absteht oder vorsteht sondern vielmehr im Sandwich geklemmt wird. Es ist weiter eine Aufgabe der vorliegenden Erfindung, eine alternative Vorrichtung oder ein alternatives System zu schaffen, das beim Injizieren einer Linse in ein Auge die vordere Haptik möglichst lange bei der Optik hält, sodass sich die Haptik möglichst erst im Auge von der Optik abfaltet und dies vorzugsweise erst wenn sich die Optik entfaltet. Diese Aufgabe soll vorzugsweise derart gelöst werden, dass damit keine zusätzlichen Teile, keine zusätzlichen Kosten und keine zusätzlichen Anwendungsschritte verbunden sind. Das System bzw. die Vorrichtung soll sowohl für voll-vorgeladene (fully-preloaded) als auch halb-vorgeladene (semi-preloaded) Linsen geeignet sein. Das System soll zudem in Verbindung mit Flügelkartuschen, insbesondere in Injektorgehäuse einlegbaren Flügelladekammern, funktionieren.

Es ist weiter eine Aufgabe der vorliegenden Erfindung, eine Vorrichtung zum einfachen Beladen mit einer intraokularen Linse zu schaffen, welche die Nachteile der beschriebenen bekannten Systeme und Methoden vermeidet. Weiter ist es eine Aufgabe der vorliegenden Erfindung, eine Vorrichtung bereitzustellen, welche eine intraokulare Linse faltet, ohne dass diese beim Falten und/oder Injizieren beschädigt wird. Im Weiteren soll eine Vorrichtung bereitgestellt werden, welche hinsichtlich der Manipulationsschritte zur Vorbereitung von Linse und Injektor optimiert ist. Insbesondere sollten möglichst wenige Manipulationsschritte nötig sein, welche nach Anlieferung von Linse und Injektor bzw. welche unmittelbar vor dem chirurgischen Eingriff an der Vorrichtung vorgenommen werden müssen. Zudem soll eine Vorrichtung bereitgestellt werden, welche mit möglichst wenigen zusätzlichen Bauteilen, im besten Fall sogar ohne jegliche zusätzliche Bauteile gegenüber bestehenden Injektoren auskommt und damit die o.g. Ziel erfüllt, ohne dabei zusätzliche Kosten zu erzeugen. Ein weiteres Ziel ist es, eine Vorrichtung zu schaffen. welche in der Anwendung lediglich kleine Inzisionen im Auge benötigt.

### ZUSAMMENFASSUNG DER ERFINDUNG

Die Erfindung ist in den Ansprüchen definiert.

Erfindungsgemäss wird die Aufgabe durch eine Vorrichtung, insbesondere eine Ladevorrichtung, zur Aufnahme einer intraokularen Linse mit einem Linsenkörper und wenigstens einer Haptik gelöst. Die Vorrichtung beinhaltet eine erste Halbschale und eine zweite Halbschale, welche an jeweils einer ersten ihrer Längsseiten durch ein erstes Gelenk gelenkig miteinander verbunden sind und relativ zueinander von einer offenen Stellung in eine geschlossene Stellung bewegt werden können, wobei die Halbschalen in der offenen Stellung eine offene Kammer zur Positionierung oder Lagerung der Linse (insb. zur Positionierung oder Lagerung der Linse in entspanntem Zustand) bilden, und die Halbschalen in der geschlossenen Stellung eine (insb. zylinderartige, mantelseitig) umschlossene Kammer zur Positionierung oder Lagerung der Linse (insb. zur Positionierung oder Lagerung der Linse in gefaltetem Zustand) und zum Ausstoss der Linse entlang der longitudinalen Erstreckung der Halbschalen bilden. Die Vorrichtung zeichnet sich vorteilhafterweise dadurch aus, dass in zumindest einer der Halbschalen eine wenigstens von der Halbschaleninnenseite her offene Aussparung ausgeformt ist, welche zur Aufnahme einer vorderen Haptik der Linse in geschlossener Stellung der Halbschalen geeignet ist.

Die Erfindung bringt den Vorteil, dass durch das Reservoir sichergestellt wird, dass die vordere Haptik einer vorgeladenen Linse mit c-loop Haptik in der Ladekammer, insbesondere bei Verwendung einer Ladekammer bestehend aus zwei durch ein Scharnier miteinander verbundenen Halbschalen, derart sicher und reproduzierbar auf die Optik positioniert wird, insbesondere im Sandwich, sodass die Haptik erst entfalten kann, wenn auch die Optik im Auge entfaltet. Insbesondere kann beim Injizieren einer Linse in ein Auge die vordere Haptik sich erst von der Optik, d.h. dem Linsenkörper, lösen, wenn die Optik beginnt sich zu entfalten.

Die im Folgenden angeführten vorteilhaften Ausführungsmerkmale führen allein oder in Kombination miteinander zu weiteren Verbesserungen der erfindungsgemässen Vorrichtung und ihrer Anwendung.

Vorteilhaft ist, dass die Aussparung in der geschlossenen Stellung der Halbschalen einen Bereich, insbesondere einen Nebenraum bildet, welcher längsseitig zur umschlossenen Kammer angeordnet ist und zweckmässigerweise derart ausgestaltet ist, dass die vordere Haptik der Linse darin aufgenommen werden kann, während die Optik der Linse in der umschlossenen Kammer positioniert ist.

Vorteilhaft ist, dass jede Halbschale mit wenigstens einer Gleitschiene ausgestattet ist, wobei die wenigstens eine Gleitschiene zur Führung des Linsenkörpers und optional des Endes der hinteren Haptik geeignet ist, Dadurch ist die Position des Linsenkörpers, d.h. der Optik, in der Vorrichtung verlässlicher vorgegeben. Die Gleitschiene dient insbesondere dem Zweck des Ausstosses der Linse aus der umschlossenen Kammer bzw. aus einem Injektor zu leiten)

Besonders vorteilhaft ist, dass zumindest eine der Halbschalen, vorzugsweise die zweite Halbschale, mit einer Auflage für die vordere Haptik, ausgestattet ist, wobei die Auflage zur Führung der vorderen Haptik, insbesondere des freien Endes der vorderen Haptik geeignet ist. Beim Laden oder zur Lagerung kann das freie Ende der vorderen Haptik im im Wesentlichen entspannten Zustand auf die Auflage gelegt sein. Diese Auflage zur Führung der vorderen Haptik ist vorzugsweise parallel zu der bzw. den Gleitschienen zur Führung des Linsenkörpers angelegt, wobei die Gleitschienen vorzugsweise tiefer in der offenen Kammer liegen als die Auflage zur Führung der vorderen Haptik. Die Auflage für die vordere Haptik kann am längsseitigen Rand der zweiten Halbschale als in die offene Kammer vorstehende Randleiste ausgeführt sein. Die Auflage ist dabei vorzugsweise als parallel zu der bzw. den Gleitschienen angeordnete durchgehende Führungsstruktur ausgeführt Eine auf der Auflage aufliegende vordere Haptik liegt damit in der offenen Kammer nicht in einer coplanaren Ebene mit der Optik sondern erhöht gegenüber der Ebene in welcher die Optik liegt. Die Auflage kann alternativ unterbrochen sein und/oder gegebenenfalls an einer düsennahen Seite der Vorrichtung als separote Halterung ausgeführt sein. Insbesondere kann die Auflage an der düsennahen Seite derart abgesenkt sein, dass die vordere Haptik, wenn sie auf der Auflage liegt, in Bezug auf die Optik coplanar liegt.

Zweckmässig sind Auflage und die Aussparung derart ausgestaltet und wirken derart zusammen, dass beim Schliessen von der offenen Stellung in die geschlossene Stellung der Halbschalen die vordere Haptik (43) sich zunehmend stärker über die Auflage hinweg und aus der sich bildenden geschlossenen Kammer erstreckt, um bei geschlossener Stellung in der Aussparung zu liegen zu kommen.

Zweckmässigerweise bildet die in der geschlossenen Stellung der Halbschalen umschlossene Kammer im Wesentlichen einen Kanal, insbesondere einen Ladekanal bzw. einen Ausstosskanal. Die umschlossene Kammer bildet im Wesentlichen einen zylinderartigen Kanal, dessen Mantelseite im Wesentlichen durch die Halbschalen definiert ist, wobei die genannte Aussparung eine Öffnung in der Mantelseite bildet. Die Aussparung zur Aufnahme der vorderen Haptik der Linse bildet im Kanal vorzugsweise eine bis zur Stirnseite des Kanals reichende seitliche Öffnung, in welcher die vordere Haptik der Linse aufgenommen werden kann.

Vorteilhaft ist, dass an jeweils einer zweiten Längsseite der beiden Halbschalen, insbesondere am jeweiligen langsseitigen Rand der Halbschalen, Flügel angeordnet sind, sodass die Halbschalen mittels der Flügel und durch Drehung um das Gelenk relativ zueinander von einer offenen Stellung in eine geschlossene Stellung bewegt werden können, wobei vorzugsweise die Aussparung zumindest in einem der Flügel fortsetzt

Bevorzugt ist, dass die Aussparung so angelegt ist, dass die Haptik beim Eingleiten in die Aussparung während des Schliessens der Halbschalen nicht wesentlich gestaucht. oder blockiert wird. Zweckmässig ist, dass die Aussparung flügelinnenseitig angelegt ist und vorzugsweise derart, dass bei geschlossener Stellung der Halbschalen die Aussparung den Massen der Haptik im Wesentlichen angepasst ist

Zweckmässig ist weiter, dass die Aussparung so angelegt ist, dass diese in von zwei im Wesentlichen parallelen Flächen (insbesondere der Innenflächen der beiden Flügel) gebildet wird, wobei die genannten parallelen Flächen (vorzugsweise im Wesentlichen gebildet durch die Flügel) in der geschlossenen Stellung der Halbschalen einen gegenseitigen Abstand aufweisen, welcher wenigstens der Dicke (bzw. dem Durchmesser) der Haptik entspricht oder diese übersteigt und vorzugsweise das fünffache oder weiter bevorzugt das doppelte der Dicke der Haptik nicht übersteigt. Vorzugsweise ist die Aussparung zwischen den im Wesentlichen parallelen Flächen mindestens so breit und tief angelegt, dass die Haptik beim Eingleiten in die Aussparung in dem in Breite und Tiefe aufgespannten Raum im Wesentlichen nicht gestaucht, geklemmt oder blockiert wird.

Optional ist es möglich, dass längsseitig an der ersten der beiden Halbschalen ein Deckelglied verschwenkbar angeordnet ist, welches in der offenen Stellung der Halbschalen die offene Kammer überdeckt und in der geschlossenen Stellung der Halbschalen im Wesentlichen ausserhalb der umschlossenen Kammer positioniert ist.

Optional kann an den Flügeln ein Verschluss, insbesondere ein Schnappverschluss, ausgebildet sein.

Optional kann an einer der Halbschalen eine Steckvorrichtung zum Einstecken in eine Aufnahmeöffnung eines Injektorgehäuses ausgebildet sein. Vorzugsweise ist die Steckvorrichtung an der zweiten Halbschale vorgesehen.

Die Vorrichtung kann einstückig sein. Herstellungstechnisch ist es von Vorteil, wenn die Vorrichtung einstückig ist und vorzugsweise aus Kunststoff besteht. Spritzgusstechnik kann in diesem Fall angewendet werden.

Die Vorrichtung kann zweckmässigerweise als Kartusche zum Einsetzen in einen Injektor, insbesondere in ein Injektorgehäuse, ausgebildet sein. Vorteilhafterweise kann die erfindungsgemässe Vorrichtung ein integrierter Teil eines Injektors sein.

Im Weiteren ist hierin offenbart ein Injektor mit einem Injektorgehäuse und einem im Injektorgehäuse längsverschiebbaren Stössel zur Verwendung mit einer als Kartusche ausgebildeten Vorrichtung wie sie oben beschrieben ist.

Weiter offenbart ist ein Injektor mit einem Injektorgehäuse mit einer darin vorgesehenen Ladevorrichtung, einer der Ladevorrichtung vorgelagerten Düse und einem im Injektorgehäuse zur Düse hin längsverschiebbaren Stössel, wobei die Ladevorrichtung mit einer Kammer ausgestattet ist, welche zwecks Ausstoss einer Linse mittels dem Stössel durchstossen werden kann, wobei die Ladevorrichtung wie oben beschrieben, insbesondere mit zusammenklappbaren Halbschalen, ausgeführt sein kann. Zweckmässigerweise zeichnet sich der Injektor dadurch aus, dass zumindest in der geschlossenen Stellung der Halbschalen düsenseitig der Halbschalen eine Faltkante angelegt bzw. vorgesehen ist, durch welche beim Vorschieben der Linse die vordere Haptik in die gefaltete Linse, insb. zwischen die Schenkel (bzw. Schenkellappen) des gefalteten Linsenkörpers, eingedrückt bzw. eingefaltet wird. Die Faltkante begrenzt die Aussparung zur Düse hin.

Die Faltkante ist vorzugsweise zwischen Ladevorrichtung und Düseneintritt einer Düse und insbesondere zwischen Aussparung der Ladevorrichtung und daran angrenzendem Düseneintritt vorgesehen. Beispielsweise ist die Faltkante am Düseneintritt ausgebildet, zweckmässigerweise dort wo der Düseneintritt einer Düse an die Ladevorrichtung und insbesondere an die Aussparung der Ladevorrichtung angrenzt. Alternativ könnte die Faltkante anstatt am Düseneintritt der Düse an zumindest einer der Halbschalen angeformt sein oder am Gehäuse, so dass sie zwischen Ladevorrichtung und Düse ausgebildet ist. Bei Ausführungen, bei welchen Düse und Ladevorrichtung einteilig ausgeführt sind, ist die Faltkante zweckmässigerweise zwischen Düsenbereich und Ladebereich angelegt.

Zweckmässig ist, dass in der geschlossenen Stellung der Halbschalen die Faltkante düsenseitig derart angelegt ist, dass beim Vorschieben der Linse die vordere Haptik in die gefaltete Linse, insb. zwischen die Schenkel des gefalteten Linsenkörpers, eingefaltet bzw. eingedrückt wird.

Weiter offenbart ist ein Verfahren zum Falten einer intraokularen Linse beinhaltend die Schritte:
- Bereitstellen einer Ladefläche mit einem vorderen, düsennahen und einem hinteren, düsernfernen Bereich, welche zumindest durch eine erste Halbschale und eine zweite Halbschale definiert ist, wobei die beiden Halbschalen über ein erstes Gelenk miteinander gelenkig verbunden sind,
- Aufbringen (insbesondere auflegen oder aufschieben) einer Linse auf die Ladefläche, indem der Linsenkörper der Linse auf die Ladefläche gebracht wird, sodass in Bezug auf den Linsenkörper eine vordere Haptik der Linse im vorderen, düsennäheren Bereich der Ladefläche positioniert ist,
- Zusammenführen der beiden Halbschalen über das erste Gelenk, indem die beiden Halbschalen durch Drehung um das erste Gelenk aufeinander zu geführt werden (d.h. insb. Zusammenklappen der Flügel), wodurch der Linsenkörper ungefähr mittig gefaltet wird, sodass ein anfänglich in seinem entspannten Zustand im Wesentlichen linsenförmiger Linsenkörper in eine Form mit zwei gegeneinander geklappten Schenkeln gepresst wird. Erfindungsgemäss zeichnet sich das Verfahren dadurch aus dass, die vordere Haptik beim Falten des optischen Linsenkörpers aus dem sich um den Linsenkörper schliessenden Raum zwischen den Halbschalen entweicht, indem das freie Ende der Haptik in eine dafür vorgesehene Aussparung in wenigstens einer der Halbschalen rutscht.

Vorteilhaft ist, dass der zwischen den sich schliessenden Halbschalen entstehende Raum im Wesentlichen zylinderförmig ist, und die Aussparung derart angelegt ist, dass die Haptik zylindermantelseitig aus dem Raum entweichen kann.

Vorteilhaft ist, dass die Linse auf der Ladefläche derart orientiert wird, dass die vordere Haptik der Linse über dem Gelenk derart zu liegen kommt, dass der Ansatz der Haptik, welcher die Haptik mit dem optischen Linsenkörper verbindet, über der ersten Halbschale und das Ende der Haptik über der zweiten Halbschale positioniert ist.

Vorteilhaft ist, dass die Linse in spannungslosem Zustand in den Hohlraum eingeführt werden kann. Das heisst, die Linse kann ohne von aussen angelegter mechanischer Spannung, insbesondere ohne Verbiegung oder Faltung der Linse, in die Vorrichtung (insbesondere per Hand) eingelegt werden.

Zweckmässigerweise liegt die Linse - insbesondere nach dem Einlegen per Hand - auf den Innenflächen der beiden Halbschalen auf. Das heisst, dass die Linse zumindest auf der Innenfläche jeder Halbschale an einem Punkt aufliegt.

Zweckmässig ist, dass jede Halbschale mit einer Auflage, z.B. als Gleitschienen ausgebildet, ausgestattet ist.

Zweckmässigerweise ist der optische Linsenkörper an seinen Rändern durch die beiden Halbschalen eingefasst und wird mit den Halbschalen zusammen, insbesondere in ungefähr gleicher Richtung, gefaltet.

Vorzugsweise beinhaltet das Verfahren im Verfahrensschritt zum Zusammenführen der beiden Halbschalen ein Zusammenführen bis zum gegenseitigen Aneinanderstossen der beiden längsseitigen Ränder der beiden Halbschalen (wobei ein Deckelglied - insoweit vorhanden - eingeklemmt wird),

Erfindungsgemäss ergibt sich, dass sich Optik und optional hintere Haptik beim Schliessen der Ladekammer absenken, während die vordere Haptik in die Aussparung geführt wird.

Im Weiteren offenbart ist ein Verfahren zum Falten einer intraokularen Linse und Ausstossen der Linse durch eine lnjektionsdüse, beinhaltend die Schritte:
- Falten der Linse, vorzugsweise nach dem vorgenannten Verfahren, wodurch der Linsenkörper ungefähr mittig gefaltet wird, sodass ein anfänglich in seinem entspannten Zustand im Wesentlichen linsenformiger Linsenkörper in eine Form mit zwei gegeneinander geklappten Schenkeln gepresst wird,
- Stossen des gefalteten optischen Linsenikörpers zur Injektionsdüse hin, wobei durch eine zunehmende Verengung in Richtung zur Injektionsdüse die gefaltete Linse zunehmend stärker zusammengedrückt wird. Das Verfahren zum Falten und Ausstossen zeichnet sich insbesondere dadurch aus, dass beim Stossen zur Injektionsdüse hin die anfänglich in der Aussparung positionierte vordere Haptik mitgezogen wird und in einen sich mit zunehmendem Vorstossen weiter verengenden Spalt zwischen den Schenkeln des gefalteten Linsenkörpers geklemmt wird. Zweckmässigerweise wird dabei die vordere Haptik aus der Aussparung heraus über eine Kante gezogen, sodass die vordere Haptik (insb. aufgrund des auf diese aufgebrachten Drucks) zwischen die Schenkel des gefalteten Linsenkörpers (41) geklemmt wird.

Die Erfindungsgemässe Vorrichtung kann in der Anwendung mit relativ kleinen Inzisionen (insbesondere bei Inzisionen mit einem Durchmesser von weniger als 2,5 mm, bevorzugt weniger als 2,2 mm, weiter bevorzugt weniger als 2 mm, weiter bevorzugt weniger als 1,5 mm) am Auge verwendet werden. Dies gelingt, weil die erfindungsgemässe Vorrichtung eine mantelartig amschliessende und somit (längsseitig) geschlossene Ladekammer für eine zu injizierende, gefaltete Linse aufweist, Die Linse ist darin auf einen besonders kleinen Querschnittsdurchmesser vorgefaltet und kann mittels einer engen Kanüle und durch eine - wie beschrieben - besonders kleine Inzision injiziert werden. Besonders vorteilhaft wird die genannte Vorrichtung mit einem verformbaren Stempel, insbesondere einem Silikonstempel (z.B. gemäss der Offenbarungsschrift WO 03/045285 A1), verwendet.

Zusätzliche Vorteile und Ziele der vorliegenden Erfindung ergeben sich aus der nachfolgenden Beschreibung.

### KURZBESCHREIBUNG DER FIGUREN

Weitere bevorzugte Ausführungen der Erfindung ergeben sich aus der nun folgenden Beschreibung anhand der Figuren. Es zeigen schematisch, in nicht massstabsgetreuen Darstellung:
- Figur 1:: eine schräge Ansicht der erfindungsgemässen Ladevorrichtung in offener Stellung mit aufgenommener intraokularer Linse;
- Figur 2:: eine Vorderansicht der erfindungsgemässen Ladevorrichtung in offener Stellung mit aufgenommener intraokularer Linse;
- Figur 3:: eine Vorderansicht der erfindungsgenässen Ladevorrichtung in geschlossener Stellung;
- Figur4:: eine Vorderansicht der erfindungsgemässen Ladevorrichtung in geschlossener Stellung mit aufgenommener intraokularer Linse;
- Figur 5:: eine schräge Ansicht der erfindungsgemässen Ladevorrichtung in geschlossener Stellung
- Figur 6:: eine schräge Ansicht einer alternativen erfindungsgemässen Ladevorrichtung in offener Stellung mit aufgenommener intraokularer Linse;
- Figur 7:: eine schräge Ansicht eines Injektors mit Düse und eingesteckter erfindungsgemässer Ladevorrichtung in geschlossener Stellung;
- Figur 8:: eine schräge Ansicht einer Anordnung der erfindungsgemässen Ladevorrichtung in geschlossener Stellung mit vorgelagertem Düsenteil (so wie die Anordnung in Fig. 7 im Injektor mit eingesetzter Ladevorrichtung oder integrierter Ladekammer enthalten ist);
- Figur 9:: ein Längsschnitt durch eine Anordnung der erfindungsgemässen Ladevorrichtung in geschlossener Stellung mit vorgelagerten Düsenteil

### DETAILIERTE BESCHREIBUNG DER FIGUREN

Im Folgenden stehen gleiche Bezugsziffern für gleiche oder funktionsgleiche Elemente (in unterschiedlichen Figuren).

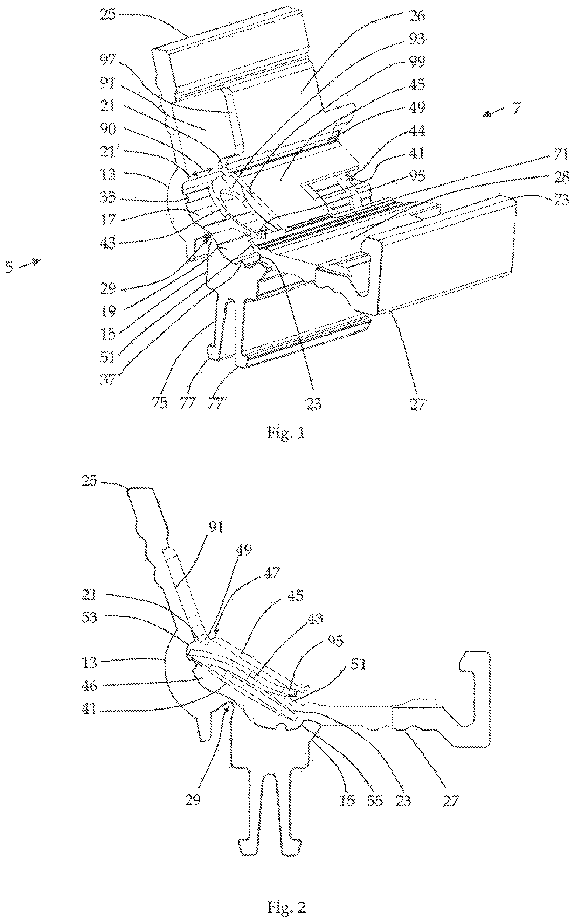

In den Fig. 1-5 ist eine erfindungsgemässe Vorrichtung in Form einer in ein Injektorgehäuse 1 einsetzbaren Kartusche 3 dargestellt. Alternativ kann die erfindungsgemässe Vorrichtung Teil eines Injektors sein bzw. fix im Injektor integriert bzw. ausgebildet sein.

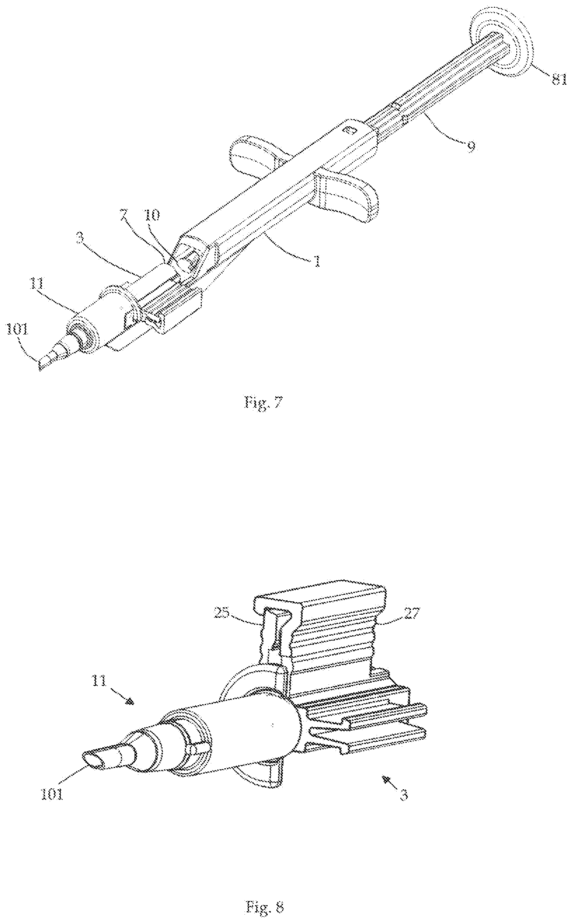

In Fig. 7 ist ein Injektor mit eingesetzter Kartusche 3 dargestellt.

In Fig. 8 ist eine Anordnung einer Kartusche 3 und einer Düse 11, wie sie in einem Injektor vorkommt, gezeigt.

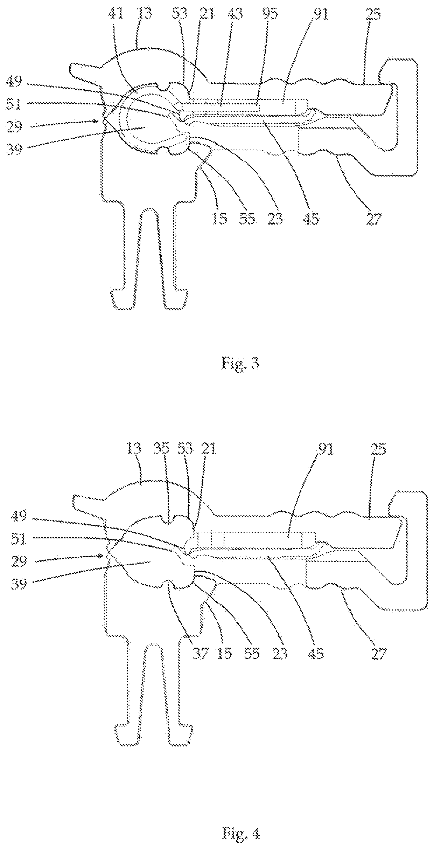

Ein Injektor ist ein Operationswerkzeug mit einem hülsenartigen Gehäuse 1 und einem im Gehäuse axial beweglich aufgenommenen Stössel 9 (Fig. 7), wobei der Stössel 9 zu einer Düse 11 hin vorschiebbar ist, welche am dorsalen Ende des Injektorgehäuses 1 ausgebildet ist und somit dem Stössel 9 vorgelagert ist. Vorzugsweise ist dem Stössel 9 ein elastischer Stempel 10 aufgesetzt. Im Mantel des Gehäuses 1 ist vorzugsweise eine Ausnehmung vorgesehen, in welche ein Linsenträger, d.h. insbesondere die hierin beschriebene Kartusche 3, derart ladbar ist, dass der Stössel 9 beim Vorschieben durch diese hindurch schiebbar ist, Die Kartusche 3 schliesst im Wesentlichen hinter der Düse 11 an. Alternativ könnte ein Injektorgehäuse mit einer integrierten Ladekammer ausgerüstet sein, wobei die integrierte Ladekammer im Wesentlichen ähnlich der hier vorgestellten Kartusche in das Gehäuse eingesetzter Form ausgeführt wäre. Der Linsenträger bzw. die Kartusche 3 besitzt einen vorzugsweise zylindrischen Ladekanal 39 (Fig. 3-5), an welchen sich axial die sich zur Spitze hin verjüngerde Injektordüse 11 (distales Ende des Linsenträgers) anschliesst (Fig. 8). Der Linsenträger bzw. die Kärtusche 3 wird derart im Injektorgehäuse 1 angelegt bzw. eingelegt und gehalten, dass der Stössel 9 fluchtend mit dem Ladekanal ist. Der Stössel 9 kann durch manuelles Anstossen am Stösselende 81 voörgeschoben werden. Beim Vorschieben des Stössels 9 dringt der Stössel 9 in den Ladekanal 39 ein und schiebt eine darin gelagerte durch die Injektordüse 11 aus, um dabei z.B. in ein Auge injiziert zu werden.

Die Kartusche 3 hat eine vorderes, d.h. düsennahes, Ende 5 und ein hinteres, d.h. düsenfernes, Ende 7 (Fig. 1, 7 und 8). In ein Injektorgehäuse 1 eingesetzt kann vom hinteren Ende 7 her der Stossel 9 in und durch die Kartusche 3 Richtung vorderes Ende 5 der Kartusche 3 und weiter in die Injektionsdüse 11 gestossen werden.

In Fig. 1 gezeigt ist eine erfindungsgemässe Ladevorrichtung, welche vorteilhafterweise als einsetzbare Kartusche 3 ausgeführt ist, welche in einen Injektor eingesetzt werden kann, wie in Fig. 7 gezeigt. Die Kartusche 3 beinhaltet zwei Halbschalen 13 und 15, welche über ein erstes Gelenk 29 miteinander gelenkig verbunden sind. Die zwei Halbschalen 13 und 15 sind insbesondere zylindersegmentartig ausgeführt und längsverlaufend gelenkig miteinander verbunden. Die zwei gelenkig miteinander verbundenen Halbschalen 13 und 15 bilden zusammen eine Doppelhalbschale. Jede Halbschale 13, 15 weist innenseitig einen offenen Halbkanal mit einer Innenfläche 17 bzw. 19 auf. Die Innenflächen 17 und 19 bilden zusammen eine Ladefläche. Die Ladefläche 17, 19 wird längsseitig von einem ersten Rand 21, welcher an der ersten Halbschale 13 ausgeführt ist, und einem zweiten Rand 23, welcher an der zweiten Halbschale 15 ausgeführt ist, begrenzt. Jede Halbschale 13, 15 weist den Rand 21 bzw. den Rand 23 auf der dem ersten Gelenk 29 fernen Seite auf. An jedem Rand 21, 23 ist vorteilhafterweise ein Flügel 25, 27 angeordnet. Die Halbschalen 13 und 15 sind über das erste Gelenk 29, welches zweckmässigerweise z.B. als Scharnier bzw. Filmscharnier ausgeführt ist, in Längsrichtung gelenkig miteinander verbunden. Die beiden Halbschalen 13 und 15 sind dadurch derart aneinandergereiht, dass die Innenflächen 17 und 19 der beiden Halbschalen 13 und 15 nebeneinander liegen, insbesondere in Längsausrichtung seitlich aneinandergrenzen (bzw. über das erste Gelenk 29 ineinander übergehen) und eine gemeinsame Ladefläche 17, 19 bilden. Längs oder längsseitig bedeutet hierbei in Ausrichtung entlang der Erstreckung oder Ausrichtung der Halbkanäle, Die Kartusche 3 kann aufgrund des Gelenks 29 von einer offenen Stellung in eine geschlossene Stellung übergeführt werden. In offener Stellung (Fig. 1 und 2) bilden die beiden Halbschalen 13 und 15 eine Art Ladefläche 17, 19, auf welche eine intraokulare Linse aufgelegt werden kann und auf welcher die Linse gegebenenfalls spannungslos gelagert werden kann. Beim Schliessen der Kartusche 3 nähern sich die längsseitigen Ränder 21 und 23 der beiden Halbschalen 13 und 15 einander an und eine auf der Ladefläche 17, 19 aufliegende Intraokularlinse wird dabei erfasst und gefaltet. In geschlossener Stellung (Fig. 3, 4 und 5) bilden die beiden Halbschalen 13 und 15 zusammen einen im Wesentlichen mantelseitig geschlossenen Kanal, d.h. den vorgenannten Ladekanal 39, welcher dazu dient die Linse in gefaltetem Zustand zwecks Injektion in ein Auge bereit zu halten.

Intraokulare Linsen bestehen im Wesentlichen aus einem optischen Linsenkörper 41 (auch Optik genannt) und einer oder mehreren Haptiken 43, 44, vorzugsweise einer ersten und einer zweiten Haptik 43, 44 (Fig. 1), welche in ihrer häufigsten Ausführungsform in der Linsenebene von der Peripherie des Linsenkörpers 41 spiralartig (insbesondere gleichgerichtet spiralartig) abstehen und federnd ausgebildet sind. Der Haptikbereich, welcher eine Haptik mit der Optik 41 verbindet, kann als Haptikansatz, z.B. 93, bezeichnet werden. Das freie Ende 95 der Haptik kann als Hapekende oder Spitze bezeichnet werden. Die Linse liegt in offener Stellung der Halbschalen 13, 15 derart auf der Ladefläche 17, 19, dass die erste Haptik 43 (inklusive Ansatz 93 und Spitze 95) in Bezug auf den Linsenkörper 41 düsenseitig positioniert ist (im Weiteren «vordere Haptik» genant), während die zweite Haptik 44 (im Weiteren «hintere Haptik» genannt) düsenfern positioniert ist.

Zur Aufnahme der vorderen Haptik 43 ist der in Längserstreckung der Halbschale 13 verlaufende Rand 21 in seiner ihrer longitudinalen Erstreckung in einem Teilbereich 21'versetzt bzw. heruntergesetzt oder anders ausgedrückt bildet einen Versatz, so dass bei geschlossener Stellung der Halbschalen 13, 15 die Innenfläche 17 der ersten Halbschale 13 einen Einbruch (bzw. eine Öffnung) 90 in der Kanalwandung aufweist, der durch die Ränder 21, 21' und 23 definiert ist. Der Versatz kann in die Flügelfläche hinein fortgesetzt sein, zum Beispiel so dass die Flügeldicke unter dem Versatz verringert ist. Damit ergibt sich in geschlossener Stellung der Halbschalen 13, 15, d.h. wenn die Ränder 21 und 23 bzw. die Flügel 25 und 27 sich am nächsten sind oder gegebenenfalls sich im Wesentlichen berühren, eine Aussparung 91 seitlich am Ladekanal 39, die sich von den Rändern 21', 23 ausgehend zwischen den Flügeln 25, 27 erstreckt. Der Einbruch 90 bzw. die Aussparung 91 sind derart ausgestaltet, dass die vordere Haptik 43 beim Falten des Linsenkörpers 41 aus dem sich bildenden Ladekanal 39 entweichen kann. Der genannte Versatz und damit der sich daraus ergebende Einbruch 90 bzw. die sich daraus ergebende Aussparung 91 in der geschlossenen Stellung sind zweckmässigerweise düsenseitig oder zumindest dasenseitennah (d.h. im vorderen Teil der Kartusche 3) angelegt, sodass die vordere Haptik 43 beim Falten der Optik durch den vom versetzten Rand 21' gebildeten Einbruch 90 (Spalte zwischen Rand 21' und 23) aus dem Kanalraum 39 in die Aussparung 91 entweichen kann.

Der Einbruch 90 bzw. die Aussparung 91 ist zweckmässigerweise wenigstens von der Halbschaleninnenseite 17, 19 her, insbesondere der umschlossenen Kammer 39 her, zugänglich, womit die Aussparung zur Aufnahme einer vorderen Haptik 43 der Linse in geschlossener Stellung der Halbschalen 13, 15 geeignet ist. Während nach Verschluss der Flügel 25, 27 (d.h. in geschlossener Stellung) die Optik 41 gefaltet in der Kammer 39 positioniert ist, ist die vordere Haptik 43 in der Aussparung 91 positioniert.

Insoweit Flügel 25, 27 an den Rändern 21, 23 anschliessen, kann sich die Aussparung 91 vom Rand 21 ausgehend im Flügel 25 fortsetzen. In der praktischen Ausführung ist der Flügel 25 in einem Teilbereich mit einer geringeren Wanddicke ausgestattet, sodass sich die Aussparung 91 an der Innenfläche 26 des Flügels 25 ergibt.

Optional ist die Aussparung 91 nicht nur zur Halbschaleninnenseite 17 hin offen sondem auch zur Düsenseite hin.

Obschon im dargestellten Ausführungsbeispiel der Einbruch 90 bzw. die Aussparung 91 in Rand 21 und Flügel 25 der ersten Halbschale 13 angelegt ist, könnte alternativ oder zusätzlich eine entsprechende Aussparung in Rand 23 und Flügel 27 angelegt sein. In einer weiteren weniger bevorzugten Alternative könnte eine funktionsähnliche Aussparung an anderer Stelle einer Halbschale 13 oder 15, zum Beispiel als durchgängiges Loch (nicht gezeigt), zweckmässigerweise in der düsennäheren Hälfte der Kartusche 3, vorzugsweise nahe dem Rand 21 oder 23, angelegt sein.

Beim Laden des Injektors liegt die entspannte Interokularlinse, insbesondere deren Optik 41, zweckmässipgerweise zwischen den längsseitigen Rändern 21, 23 vorzugweise auf einer Leitstruktur der Ladefläche 17,19 auf, wobei die Leitstruktur hier zum Beispiel aus Gleitschienen 35, 37 besteht, weiche auf der Ladefläche 17, 19 längsgerichtet ausgebildet sind. Die Gleitschienen 35, 37 sind insbesondere als Rippen ausgeführt.

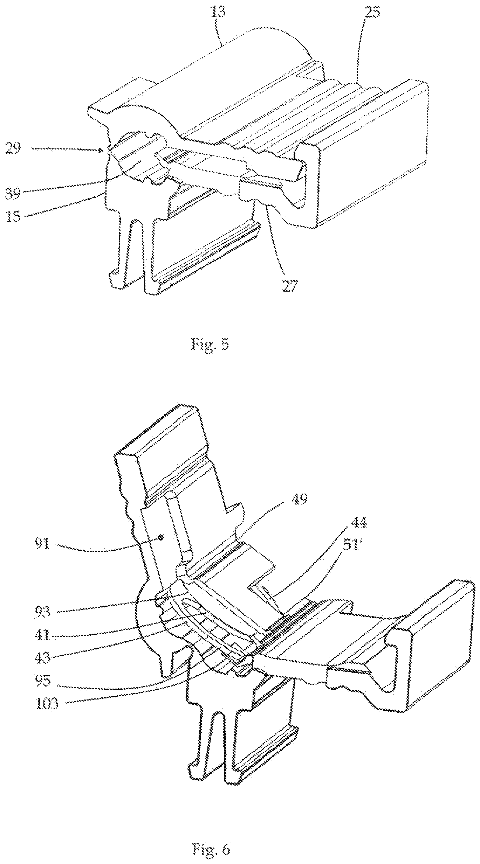

Die beiden längsseitigen Ränder 21, 23 sind zweckmässigerweise mit längs gerichteten Leisten 49, 51 (wobei die Leiste 51 im Weiteren auch Führungsschiene genannt wird) bestückt. Die Leisten 49, 51 sind beide zweckmässigerweise quer zur Längsrichtung gekrümmt und gegebenenfalls mit sich verjüngenden Längsseiten ausgeführt. Die Krümmung der Leisten 49, 51 ist dergestalt, dass sich die beiden Leisten bei geschlossener Kartusche zu einer halbkreisartigen Ausbauchung ergänzen, welche sich in den geschlossenen Kanal 39 erstreckt. Die Leisten 49, 51 bilden in seitlicher Fortführung der Innenflächen 17, 19 am jeweiligen längsseitigen Rand 21, 23 eine Gegenfläche zur jeweiligen Innenfläche 17, 19, wodurch beidseitig eine Art innenseitige Nut 53, 55 am jeweiligen långsseitigen Rand 21, 23 entsteht (Fig. 3). Leisten 49, 51 bzw. insbesondere die dadurch definierten Nuten 53, 55 (und optional die Gleitschienen 35, 37) können als Leitsystem zur Einführung und Faltung einer Linse, insbesondere des Linsenkörpers 41 zusammenwirken. Die Leisten 49,51 können in Längsrichtung durchgehend (wie in Fig. 1 gezeigt) oder unterbrochen (Fig. 6) ausgebildet sein. Das Leitsystem ist zweckmässigerweise parallel zur Längserstreckung der Halbschalen 13, 15 ausgerichtet. Die Leiste 51 an der zweiten Halbschale 15 ragt vorzugsweise genügend weit in die Halbschale 15 ein, dass die Spitze 95 der vorderen Haptik 43 auf die Leiste 51 gelegt werden kann, während gleichzeitig der Linsenkörper beidseitig unter den Leisten 49, 51 auf den Innenflächen 17, 19, insb. auf den Gleitschienen 35, 37, aufliegt.

Der Ablauf des Ladens der Linse in der erfindungsgemässen Vorrichtung bzw. Ladekammer läuft zum Beispiel wie folgt ab: Die Linse wird in die Ladekammer geladen, indem die Optik 41 unter der Leiste bzw. Führungsschiene 51 in die vorgeladene Position vorgeschoben wird. Dabei können die Haptiken bereits unter leichter Spannung etwas vorgefaltet werden. Dabei werden die Haptiken vorzugsweise in Richtung der Optik vorgefaltet Sie können aber auch entspannt vorgeladen werden und im weiteren Ladeverlauf über den Silikonstempel 10 des Kolbens durch den Endanwender vorgefaltet werden. Die vordere Haptik 43 wird anders als bei gängigen Ladekammern (wie z.B. in WO 2015/070358 A2) dabei auf die Führungsschiene 51 aufgelegt, welche vorzugsweise derart ausgebildet ist, insbesondere tief genug ist, damit die Haptik nicht von alleine herunterfallen kann. Beispielsweise ist die Führungsschiene 51 durchgehend und erstreckt sich im Wesentlichen in einer Geraden, so dass die Haptik 43 durch das Anheben auf die Führungsschiene 51 etwas über die Erstreckungsebene des Linsenkörpers 41 hinaus ungehoben wird.

Wenn alternativ die vordere Haptik 43 in derselben Ebene wie die Optik 41 zu liegen kommen soll, dann ist die Führungsschiene in einem vorderen Bereich 103 (d.h. düsenseitig) tiefer liegend als im hinteren Bereich (d.h. stosselseitig) bzw. entsprechend abgesenkt angelegt. Der Bereich 103 kann dabei optional als separate Ablage ausgeführt sein, welche insbesondere von einer Leiste 51' getrennt ist und gegebenenfalls von der Iongitudinalen Achse der Leiste versetzt angeordnet ist.

In Fig. 6 ist eine alternative Ladevorrichtung gezeigt, bei welcher düsenseitig ein derartiger Bereich 103 für die vordere Haptik 43 tiefer gesetzt ist als die durch die Leiste 51 definierte Auflagefläche in Fig. 1. Dies hat den Vorteil, dass die vordere Haptik 43 und die Optik 41 co-planar lagern, insoweit Gleitschiene 37 für die Optik 41 und die separate Auflagefläche 103 für die vordere Haptik 43 entsprechend aufeinander abgestimmt ausgebildet sind. Je nach Linsenmaterial kann dies insbesondere für eine Langzeitlagerung vonVorteil sein.

Während zum Beispiel die Innenflächen 17, 19, insb. die Gleitschienen 35, 37, als Auflageflächen für die Optik 41 im entspannten Zustand dienen (Fig. 1), kann die Leiste 51 als Auflagefläche für die vordere Haptik 43 im entspannten Zustand dienen. Beim Schliessen der Ladekammer drücken die Leisten 49 und 51 auf die darunter liegende Optik und hintere Haptik und bewirken, dass die Optik und hintere Haptik nach unten falten (d.h. zum ersten Gelenk 29 hin durchbiegen, siehe Fig. 3), während sich die vordere Haptik 43, welche auf der Leiste 51 aufliegt, durch den seitlichen Einbruch 90 bzw. in die seitliche Aussparung 91 in dem sich bildenden Ladekanal streckt.

Die Auflagefläche für die vordere Haptik 43 auf der Leiste 51 der zweiten Halbschale 15 und der Randversatz 21', der den Einbruch 90 definiert, bzw. die Aussparung 91 der ersten Halbschale 13, sind derart aufeinander abgestimmt, dass beim Schliessen der beiden Halbschalen 13, 15 zur Bildung des geschlossenen Kanals 39 die vordere Haptik 43 über die Leiste 51 (oder gemäss alternativer Ausführung über die separate Auflagefläche 103) in den Einbruch 90 und somit die Aussparung 91 gelangt.

Die hier dargestellte Leiste 51 hat einen Multifunktionalen Zweck. Wie die Leiste 49 dient sie mit dieser zusammen als Leitstruktur zum Einschieben der Linse und als Falthilfe, indem sie beim Schliessen der Flügel 25, 27 die Optik 41, welche unter den Leisten 49, 51 liegt, zwingen nach unten zum ersten Gelenk 29 hin zu biegen. Zudem dient die Leiste 51 als Führung der vorderen Haptik 43, zum einen beim Einschieben der Linse in die Ladevorrichtung, wobei das vordere Ende der vorderen Haptik 43 auf der Leiste in deren Längsrichtung eingeschoben wird, und zum anderen beim Schliessen, wobei die vordere Haptik quer über die Leiste 51 hinweg aus der sich schliessenden Kammer 36 geführt wird.

Früher war es wichtig, dass die Haptiken nicht auf der Führungsschiene zu liegen kommen, da sie dann beim Schliessen zwischen den Flügeln einklemmten und beim Vorschieben der Linsen abrissen. Gemäss der hier vorliegenden Offenbarung ist jedoch gewollt, dass die vordere Haptik 43 auf der Führungsschiene 51 liegt. Wenn die Flügel 25, 27 geschlossen werden, faltet sich die Linse bzw. deren Körper 41 zu einem "U". Die vordere Haptik 43 kann dabei zwischen die Flügel 25, 27 gleiten, wo aufgrund der Aussparung 91 ein Hohlraum geschaffen ist, in welchem die vordere Haptik 43 im Wesentlichen frei (d.h. ungeklemmt) zu liegen kommt. Wenn die vordere Haptik 43 frei liegt, wird diese, sobald die Optik 41 vorgeschoben wird, hinterher gezogen. Dabei zieht sie sich der Länge nach aus dem Hohlraum in die U-förmig gefaltete aber nach oben hin noch geöffnete Optik 41 (deshalb "U"-Form). Je weiter die Linse aus der Ladekammer in Richtung Düse 11 vorgeschoben wird, umso mehr schliesst sich das "U" im enger werdenden Durchgang und die vordere Haptik 43 wird zwischen den Schenkeln der U-förmig eingefalteten Optik 41 eingeschlossen.

Wie in Fig. 1 dargestellt, ist bevorzugt, dass die Aussparung 91 (d.h. der Einbruch 90 bzw. der Einbruch 90 mit Aussparung 91) an der einen Halbschale (hier an der ersten Halbschale 13) ausgebildet ist, während an einem korrespondierenden Bereich (d.h. in einem in longitudinaler bzw. axialer Erstreckung der Ladevorrichtung übereinstimmendem Bereich) der anderen Halbschale (hier der zweiten Halbschale 15) die Leiste 51 ausgebildet ist. Damit steht dem Einbruch 90, der die kammerseitige Öffnung zur Aussparung 91 hin bildet, ein Bereich der Leiste 51 (hier insbesondere der vordere bzw. im Wesentlichen düsenseitige Bereich der Leiste 51) diesem gegenüber.

Nach Austritt der Linse in das Auge, öffnet sich die bis anhin gefaltete Optik 41 und gibt die Haptik 43 wieder frei.

Der hier beschriebene Faltvorgang mit kontrolliertem Einklemmen der vorderen Haptik 43 in der gefalteten Optik 41 resultiert in einer gegenüber dem Faltvorgang in herkömmlichen Vorrichtungen mit Klapp- bzw. Flügelkammern verzögerten Freigabe der vorderen Haptik 43 beim Entfalten, sodass die vordere Haptik 43 erst im Auge freigegeben wird, wenn sich die Optik 41 entfaltet. Aufgrund der Bereitstellung einer Ladekammer mit Einbruch 90 im der Wand 17 des Ladekanals 39 und einer vom Einbruch 90 ausgebenden Aussparung 91 längsseitig des Ladekanals 39 ist der gesamte Faltvorgang (Faltung und Entfaltung) geändert, insbesondere ohne dass hierfür weitere Bauteile nötig sind.

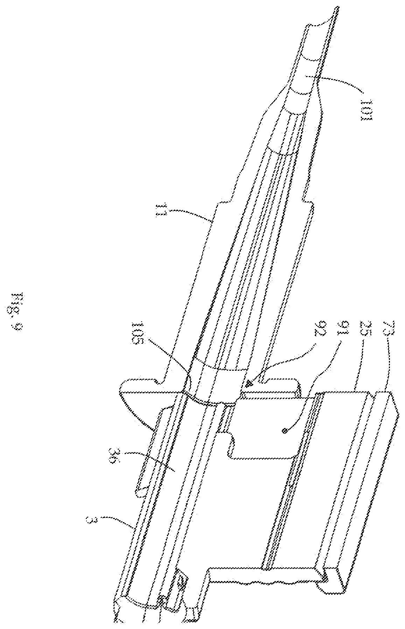

In Fig. 8 ist eine Anordnung der Kartusche 3 mit direkt vorgelagerter Düse 11 dargestellt. In Fig. 9 ist ein Längsschnitt durch eine derartige Anordnung dargestellt. Im Längsschnitt ist der Ladekanal 36 der Kartusche 3 zu erkennen, an welchen der Düsenkanal 101 anschliesst, Ladekanal 36 und Düsenkanal 101 bilden einen Durchgang für die Linse, welche im Ladekanal vorgefaltet wird und beim Durchstossen vom Düseneintritt 105 bis zum Düsenaustritt 107 hin im enger werdenden Düsenkanal 101 sukzessive stärker gefaltet und gepresst wird. Die Aussparung 91 ist seitlich des Ladekanals 36 angelegt.

Der Übergang vom Ladekanal 36 zum Düsenkanal 101 weist im Bereich der Aussparung 91 eine Kante 92 auf. Die Kante 92 bildet eine räunilich Begrenzung zwischen Aussparung 91 und Düsenkanal 101. Insbesondere bildet der Düsereintritt 105 (d.h. der Eingang in den Düsenkanal) im Bereich der Aussparung 91 eine begrenzende Kante 92, über welche beim Vorstossen einer Linse die in die Aussparung 91 gestreckt eingelagerte vordere Haptik der Linse gezogen wird. Die Kante 92 ist zweckmässigerweise im Wesentlichen quer zur Längserstreckung der Halbschalen bzw. quer zur Injektions- bzw. Ausstossrichtung ausgerichtet. Mittels dieser Kante 92 wird dabei die vordere Haptik 43 beim Vorschieben der Linse (insbesondere beim Vorschieben aus der Ladekammer 39 hin zur Düse 11) in das noch teilweise geöffnete "U" bzw. zwischen die Linsenschenkel der gefalteten Linse eingedrückt und eingefaltet, was beim weiteren Vorrücken der immer stärker gefalteten Linse zu einer Sandwichfaltung führt. Die Kante 92 zwischen Düsenkanal 101 und Aussparung 91 kann daher auch als Paltkante bezeichnet werden.

An den Flügeln 25, 27 ist ein Verschluss 73, insbesondere ein Schnappverschluss, ausgebildet.

An der Halbschale 15 ist eine Steckvorrichtung 75 ausgebildet. Diese Steckvorrichtung 75 dient zum Einstecken in eine Öffnung eines Injektorgehäuses 1. Als Blockiermittel dienen beispielsweise Federbeine 77, 77' mit Widerhaken. Dadurch wird nach dem Einstecken der Kartusche 3 in das Injektorgehäuse 1 eine feste Verbindung zwischen diesen Teilen geschaffen.

Der in Fig. 7 dargestellte Injektor besteht im Wesentlichen aus dem Injektorgehäuse 1 und dem in diesem verschiebbaren Stössel 9 zum Transportieren und Ausstossen der Linse. Bei der gezeigten Ausführungsform stellen die Kartusche 3 und ggf. die Injektordüse 11 separate in das Injektorgehäuse 1 einsetzbare Teile dar, sie können jedoch auch als ein Teil ausgebildet sein. Der Stössel 9 ist in einer Ausgangsstellung im Injektorgehäuse gelagert, vorzugsweise arretiert, sodass er beim Einsetzen der Kartusche 3 nicht im Weg ist. Durch betätigen des Kolbens am Kolbenende 81 kann eine eingelegte Linse durch Anstossen per Stössel 9 ausgestossen werden.

Zusammenfassend kann folgendes festgehalten werden:
Eine Vorrichtung zum Falten einer intraokularen Linse weist zwei durch ein Scharnier 29 verbundene Halbschalen 13, 15 auf, welche z.B. mittels an den Halbschalen 13, 15 ausgebildeten Flügeln 25, 27 relativ zueinander bewegt und gegeneinander geschlossen werden können, um eine eingelegte intraokulare Linse zu falten und gleichzeitig in einem Ladeträgerkanal, welcher sich durch das Schliessen der Halbschalen 13, 15 bildet, ausstossbereit zu halten. Zwischen den gegeneinander geschlossenen Rändern 21, 23 der Halbschalen 13, 15 ist auf der düsennahen Seite eine Aussparung 91 ausgebildet, in welche die vordere Haptik 43 beim Falten der Optik 41 weicht. Die Kante 92, welche düsenseitig vor der Aussparung 91 angelegt ist und derart ausgeführt ist, dass beim Ausstoss der Linse aus der Ladekammer, die vordere Haptik 43 aus der Aussparung 91 heraus und über die Kante 92 gezogen wird und aufgrund des dabei entstehenden Drucks auf die vordere Haptik 43, die Haptik 43 im Sandwich zwischen die gefalteten aber nach oben (zur Aussparung hin) offenen Optikschenkel eingefaltet wird. Dadurch ist es nun möglich, vorgeladene Linsen auch per Flügelkartusche (dann insbesondere durch Zusammenklappen per Flügel) oder allgemeiner per Klappkartusche zwecks Injektion in ein Auge in eine Sandwichfaltung zu bringen, was dazu führt, dass beim Durchstoss durch die Düse die Entfaltung der Haptik erst mit der Entfaltung der Optik erfolgt (d.h. nicht vor der Entfaltung der Optik). Auch ermöglicht diese Vorrichtung die vorgenannte Form der vorderen Haptikfaltung, selbst im Falle einer vom restlichen Injektor separat gelagerten Ladekammer.

Um ein Einklemmen der Linse und insbesondere ihrer Haptik 43, 44 beim Schliessen der Halbschalen 13, 15 zu vermeiden, kann die Kartusche 3 mit einem Deckelglied 45 ausgestattet sein, wie dies in WO 2015/0730358 A2 ausgeführt ist. Ein längsseitig flügelseitig an der ersten der beiden Halbschalen verschwenkbar angeordnetes Deckelglied dient dazu, eine interokulare Linse abzudecken, welche in der offenen Stellung der Halbschalen zwischen die Halbschalen eingelegt wird. Das Deckelglied überdeckt somit eine von den Halbschalen gebildete offene Kammer längsseitig und schützt gleichzeitig die Linse bzw. hält diese auf Position. Beim Schliessen der beiden Halbschalen, rutscht das Deckelglied über den Rand der zweiten Halbschale, sodass sobald die beiden Halbschalen in geschlossener Stellung sind, das Deckelglied im Wesentlichen ausserhalb der von den beiden Halbschalen definierten Kammer positioniert ist. Das Deckelglied ist (und vorzugsweise bleibt) in offener Stellung sowie in geschlossener Stellung an der ersten Halbschale befestigt. Dieses System hat den Vorteil, dass das Risiko, dass die Haptik der Linse beim Falten einklemmt, reduziert ist.

Das Deckelglied 45 ist am längsseitigen Rand 21 der ersten Halbschale 13 beweglich insbesondere verschieb- oder klappbar (ähnlich einer einflügeligen Schwingtür), angeordnet bzw. befestigt. Das Deckelglied 45 ist vorteilhafterweise als Deckelplatte ausgeführt, insbesondere als plane, formstabile Deckelplatte. In einer offenen Stellung der Kartusche 3 überspannt das Deckelglied 45 die Ladefläche 17, 19 vom längsseitigen Rand 21 der ersten Halbschale 13 bis zum längsseitigen Rand 23 der zweiten Halbschale 15.

Das Deckelglied 45 ist am längsseitigen Rand 21 der ersten Halbschale 13 vorteilhafterweise über ein zweites Gelenk 47 beweglich fixiert. Zweckmässigerweise ist das Gelenk 47 ein Scharnier, insbesondere ein Filmscharnier, und ist als Biegerille bzw. Falzbereich ausgestaltet.

Die Drehachsen des ersten und zweiten Gelenks 29 und 47 sind vorzugsweise parallel zueinander ausgerichtet.

Das Deckelglied 45 schliesst zumlängsseitigen Rand 23 hin aufgrund seiner Schwerkraft und/oder einer Federkraft im zweiten Gelenk 47. Da das Deckelglied 45 selbsttragend (d.h. genügend steif) ist, bildet sich in offener Stellung der Kartusche 3 eine gedeckte Kammer 46 zwischen erster Halbschale 13, zweiter Halbschale 15 und Deckelglied 45. Das zweite Gelenk 47 ist vorzugsweise an einer vom längsseitigen Rand 21 der ersten Halbschale 13 abstehenden Leiste 49 angeordnet. Auf der Gegenseite, d.h. am längsseitigen Rand 23 der zweiten Halbschale 15 ist vorzugsweise eine zweite abstehende Leiste 51 angeformt, welche bei offener Kartusche 3 als Auflage für das Deckelglied 45 dient. Die sich vorzugsweise verjüngende Längsseite der Leiste 49 geht zweckmässigerweise in ein Filmscharmier über.

Die Leiste 49 ist kammeraussenseitig vorzugsweise mit einer konkaven Biegerille ausgebildet. Die Biegerille weist insbesondere eine linienförmige Stoffverdrängung in Längsrichtung der ersten Halbschale 13 auf, wodurch eine Biegefähigkeit des Materials erzeugt wird. Dadurch kann das Deckelglied 45 gelenkig zum ersten Flügel 25 hin geklappt werden. Die Biegerille funktioniert insbesondere als Filmscharnier.

Der längsseitige Rand 23, d.h. insbesondere die Leiste 51, an der zweiten Halbschale 15 und das freie Ende 58 des Deckelglieds 45 sind derart ausgestaltet, dass beim Zudrücken der beiden Flügel 25, 27 (d.h. beim Zusammenbringen der Flügelgriffe per Hand) das Deckelglied 45 bzw. dessen freies Ende 58 angestossen wird und der Flügelfläche 28 entlang gleitet. Die Leiste 51 kann somit eine Art Deckelgliedauflage bilden.

In geschlossener Stellung der Kartusche 3 (Fig. 3) liegt das Deckelglied 45 im Wesentlichen ausserhalb der aus der Kammer 46 gebildeten umschlossenen Kammer 39 und vorzugsweise zwischen den Flügeln 25, 27.

Beim Schliessvorgang der Kartusche 3 indem per Hand die Flügel 25, 27 der Kartusche 3 zusammengeführt werden, rutscht das Deckelglied 45 von seiner Raststellung am Rand 23 auf die innenseitige Flügelfläche 28 des zweiten Flügels 27 und dieser Flügelfläche 28 entlang aus dem sich schliessenden Hohlraum 46 bzw. aus dem sich bildenden Hohlraum 39 heraus.

Insbesondere Zeichnet sich die Vorrichtung in Bezug auf das Deckelglied durch die folgenden Eigenschaften aus
- dass langsseitig an der ersten der beiden Halbschalen 13 ein Deckelglied 45 verschwenkbar angeordnet ist, welches in der offenen Stellung die offene Kammer 46 überdeckt und in der geschlossenen Stellung im Wesentlichen ausserhalb der umschlossenen Kammer 39 positioniert ist.
- dass das Deckelglied düsenseitig einen Randverlauf 99 aufweist, welcher gegenüber einer düsenseitigen Stirnseite der Halbschalen 13, 15 rückversetzt ist, vorzugsweise derart rückversetzt, dass bei eingelegter Linse die vordere Haptik der Linse vom Deckelglied nicht gedeckt ist, während die Optik der Linse vom Deckelglied gedeckt ist.
- dass das Deckelglied 45 gegenüber einer düsenseitigen Stirnseite der Halbschalen 13, 15 derart weit rückversetzt ist, dass bei Einlage einer Linse die vordere Haptik 43 vom Deckelglied nicht gedeckt ist während die Optik 41 gedeckt ist.
- dass das Deckelglied 45 plattenförmig ausgebildet ist,
- dass das Deckelglied 45 am längsseitigen Rand 21 der ersten Halbschale 13 verschwenkbar angeordnet ist,
- dass das Deckelglied 45 über ein zweites Gelenk 47, welches z.B. als Scharnier, insbesondere als Filmscharnier, ausgebildet ist, mit der ersten Halbschale 13 verbunden ist.
- dass eine Biegerille auf der der Innenfläche 17 der ersten Halbschale 13 abweisenden Deckelgliedfläche ausgebildet ist.
- dass am längsseitigen Rand 23 der zweiten Halbschale 15 eine Deckelgliedauflage 51 ausgebildet ist.
- dass beim Schliessen der beiden Halbschalen 13, 15 das Deckelglied 45 über den längsseitigen Rand 23 der zweiten Halbschale 15 aus der sich schliessenden Kammer 46 hinaus rutscht.
- dass in der geschlossenen Stellung der Halbschalen 13, 15 das Deckelglied 45 im Wesentlichen ausserhalb der umschlossenen Kammer 39 zwischen den Flügelgriffen 25, 27 positioniert ist,
- dass die Aussparung 91. am ersten Flügel 25 einen Randverlauf 97 aufweist, welcher in geschlossener Stellung im Wesentlichen dem düsenseitigen Randverlauf 99 des Deckelglieds 45 folgt oder (im Vergleich zum düsenseitigen Randverlauf 99 des Deckelgliede 45) weiter von *der* düsenseitigen Stimseite der Halbschalen 13, 15 rückversetzt ist,
- dass in geschlossener Stellung das Deckelglied 45 im Wesentlichen ausserhalb der umschlossenen Kammer 39 und vorzugsweise zwischen den Flügeln 25, 27 liegt.
- dass das Deckelglied 45 selbsttragend ausgeführt ist,
- dass beinhaltend im Verfahrensschritt zum Zusammenführen der beiden Halbschalen 13, 15 ein Zusammenführen bis zum gegenseitigen Aneinanderstossen der beiden längsseitigen Ränder 21, 23 der beiden Halbschalen 13, 15 das Deckelglied 45 - insoweit vorhanden - eingeklemmt wird.

Während vorstehend spezifische Ausführungsformen beschrieben wurden, ist es offensichtlich, dass unterschiedliche Kombinationen der aufgezeigten Ausführungsmöglichkeiten angewendet werden können, insoweit sich die Ausführungsmöglichkeiten nicht gegenseitig ausschliessen.

Während die Erfindung vorstehend unter Bezugnahme auf spezifische Ausführungsformen beschrieben wurde, ist es offensichtlich, dass Änderungen, Modifikationen, Variationen und Kombinationen ohne vom Erfindungsgedanken abzuweichen gemacht werden können.

### BEZUGSZEICHENLISTE

- 1: Injektorgehäuse
- 3: Kartusche
- 5: vordere Stirnseite (bzw. vorderes Ende) der Kartusche
- 7: hintere Stirnseite (bzw. hinteres Ende) der Kartusche
- 9: Stössel (auch Kolben genannt)
- 10: Stempel
- 11: Injektionskanüle bzw. -düse
- 13: erste Halbschale
- 15: zweite Halbschale
- 17: erste Innenfläche, d.h. Innenfläche der ersten Halbschale
- 19: zweite Innenfläche, d.h. Innenfläche der zweiten Halbschale
- 21: längsseitiger Rand der Ladefläche an der ersten Halbschale, d.h. erster längsseitiger Rand
- 21': versetzter Randbereich an der ersten Halbschale (Teilbereich des längsseitigen Rands 21)
- 23: längsseitiger Rand der Ladefläche an der zweiten Halbschale, d.h. zweiter längsseitiger Rand
- 25: erster Flügel
- 26: Flügelfläche des ersten Flügels
- 27: zweiter Flügel
- 28: Flügelfläche des zweiten Flügels
- 29: erstes Gelenk, insbesondere Filmscharnier
- 35: erste Gleitschiene
- 37: zweite Gleitschiene
- 39: umschlossene Kammer, Ladekanal ausgebildet als geschlossener Kanal
- 41: optischer Linsenkörper, auch Linsenkörper oder Optik genannt
- 43: vordere Haptik
- 44: hintere Haptik
- 45: Deckelglied
- 46: offene Kammer, ggf. mit Deckelglied gedeckt
- 47: zweites Gelenk, insbesondere Filmscharnier
- 49: erste Randleiste, d.h. Randleiste am längsseitigen Rand der ersten Halbschale
- 51, 51': zweite Randleiste, d.h. Randleiste am längsseitigen Rand der zweiten Halbschale, auch Führungsschiene genannt.
- 53: erste Nut
- 55: zweite Nut
- 58: freies Ende des Deckelglieds
- 71: freies Ende des Deckelglieds
- 73: Verschluss
- 75: Steckvorrichtung
- 77, 77': Federbeine
- 81: Stösselende (Kolbenende)
- 90: Einbruch in Innenwand bei geschlossener Stellung der Halbschalen
- 91: Aussparung
- 92: Kante bzw. Faltkante
- 93: Ansatz der Haptik, welcher die Haptik mit dem optischen Linsenkörper verbindet, hier insbesondere Ansatz der vorderen Haptik
- 95: Ende (Spitze) der Haptik, insbesondere auch freies Ende der Haptik genannt
- 97: stimmseitiger Randverlauf der Aussparung im Flügel
- 99: stirnseitiger Randverlauf des Deckelglieds
- 101: Düsenkanal
- 103: alternative, separate Auflagefläche für vordere Haptik
- 105: Düseneintritt
- 107: Düsenaustritt

## Patentansprüche

1. Vorrichtung, insb. Ladevorrichtung, zur Aufnahme und zum Falten einerintraokularen Linse mit einem Linsenkörper und wenigstens einer Haptik, die Vorrichtung beinhaltend eine erste Halbschale (13) und eine zweite Halbschale (15), welche an jeweils einer ersten ihrer Längsseiten durch ein erstes Gelenk (29) gelenkig miteinander verbunden sind und relativ zueinander von einer offenen Stellung in eine geschlossene Stellung bewegt werden können und deren Innenflächen (17, 19) eine Ladefläche bilden, wobei
- die Halbschalen (13, 15) in der offenen Stellung eine offene Kammer (46) zur Positionierung oder Lagerung der Linse in entspanntem Zustand bilden, und
- die Halbschalen (13, 15) in der geschlossenen Stellung eine umschlossene Kammer (36) zur Positionierung oder Lagerung der Linse in gefaltetem Zustand und zum Ausstoss der Linse entlang der longitudinalen Erstreckung der Halbschalen (13, 15) bilden, **dadurch gekennzeichnet, dass**
in zumindest einer der Halbschalen (13, 15) eine wenigstens von der Halbschaleninnenseite her offene Aussparung (91) ausgeformt ist, welche zur Aufnahme einer vorderen Haptik (43) der Linse in geschlossener Stellung der Halbschalen (13, 15) geeignet ist.

2. Vorrichtung nach dem vorangehenden Anspruch, **dadurch gekennzeichnet, dass** die Aussparung (91) in der geschlossenen Stellung der Halbschalen (13, 15) einen Bereich, insbesondere einen Nebenraum bildet, welcher längsseitig zur umschlossenen Kammer (36) angeordnet ist und derart ausgestaltet ist, dass die vordere Haptik der Linse darin aufgenommen werden kann, während die Optik der Linse in der umschlossenen Kammer positioniert ist.

3. Vorrichtung nach einem der vorangehenden Ansprüche, **dadurch gekennzeichnet, dass** zumindest eine der Halbschalen (13, 15), vorzugsweise die zweite Halbschale (15), mit einer Auflage (51, 103) für die vordere Haptik (43), ausgestattet ist, wobei die Auflage (51, 103) zur Führung der vorderen Haptik, insbesondere des freien Endes der vorderen Haptik (95) geeignet ist.

4. Vorrichtung nach dem vorangehenden Anspruch 3, **dadurch gekennzeichnet, dass** die Auflage (51, 103) **und die** Aussparung (91) derart ausgestaltet sind und derart zusammenwirken, dass beim Schliessen von der offenen Stellung in die geschlossene Stellung der Halbschalen (17, 19) die vordere Haptik (43) sich zunehmend stärker über die Auflage (51, 103) hinweg und aus der sich bildenden geschlossenen Kammer (36) erstreckt um bei geschlossener Stellung in der Aussparung (91) zu liegen zu kommen.

5. Vorrichtung nach einem der vorangehenden Ansprüche, **dadurch gekennzeichnet, dass** an jeweils einer zweiten Längsseite der beiden Halbschalen (13, 15), Flügel (25, 27) angeordnet sind, sodass die Halbschalen (13, 15) mittels der Flügel 25, 27) und durch Drehung um das Gelenk (29) relativ zueinander von einer offenen Stellung in eine geschlossene Stellung bewegt werden können, wobei vorzugsweise die Aussparung (91) zumindest in einem der Flügel (25, 27) fortsetzt.

6. Vorrichtung nach einem der vorangehenden Ansprüche, **dadurch gekennzeichnet, dass** jede Halbschale (13, 15) mit wenigstens einer Gleitschiene (35, 37) ausgestattet ist, wobei die wenigstens eine Gleitschiene zur Führung des Linsenkörpers und optional des Endes der hinteren Haptik (44), geeignet ist.

7. Vorrichtung nach einem der vorangehenden Ansprüche, **dadurch gekennzeichnet, dass** längsseitig an der ersten der beiden Halbschalen (13) ein Deckelglied (45) verschwenkbar angeordnet ist, welches in der offenen Stellung die offene Kammer (46) überdeckt und in der geschlossenen Stellung im Wesentlichen ausserhalb der umschlossenen Kammer (39) positioniert ist.

8. Vorrichtung nach einem der vorangehenden Ansprüche, **dadurch gekennzeichnet, dass** diese als Kartusche zum Einsetzen in einen Injektor, insbesondere in ein Injektorgehäuse (1), ausgebildet ist.

9. Injektor mit einem Injektorgehäuse (1) mit einer darin vorgesehenen Ladevorrichtung (3) gemäss einem der vorhergehenden Ansprüche 1-8, einer der Ladevorrichtung (3) vorgelagerten Düse (11) und einem im Injektorgehäuse (1) zur Düse (11) hin langsverschiebbaren Stössel (9), wobei die umschlossene Kammer (36) zwecks Ausstosses einer Linse mittels dem Stössel (9) durchstossen werden kann,
wobei zumindest in der geschlossenen Stellung der Halbschalen (13, 15) düsenseitig der Halbschalen (13, 15) eine Kante (92) angeordnet ist, durch welche beim Vorschieben der Linse die vordere Haptik in die gefaltete Linse, insb. zwischen die Schenkel des gefalteten Linsenkörpers, eingedrückt bzw. eingefaltet wird.

10. Injektor nach dem vorangehenden Anspruch 9, **dadurch gekennzeichnet, dass** die Faltkante (92) die Aussparung (91) zur Düse hin begrenzt, insbesondere vorzugsweise an einem Düseneintritt (105) ausgebildet ist.

11. Verfahren zum Falten einer intraokularen Linse in einer Vorrichtung beinhaltend die Schritte:
- Bereitstellen einer Ladefläche (17, 19) mit einem vorderen und einem hinteren Bereich, welche zumindest durch eine erste Halbschale (13) und eine zweite Halbschale (15) definiert ist, wobei die beiden Halbschalen (13, 15) über ein erstes Gelenk (29) miteinander gelenkig verbunden sind,
- Aufbringen einer Linse auf die Ladefläche (17, 19), indem der Linsenkörper (41) der Linse auf die Ladefläche (17, 19) gebracht wird, sodass in Bezug auf den Linsenkörper (41) eine vordere Haptik der Linse im vorderen Bereich der Ladefläche positioniert ist,
- Zusammenführen der beiden Halbschalen (13, 15) über das erste Gelenk (29), indem die beiden Halbschalen (13, 15) durch Drehung um das erste Gelenk (29) aufeinander zu geführt werden, wodurch der Linsenkörper (41) ungefähr mittig gefaltet wird, sodass ein anfänglich in seinem entspannten Zustand im Wesentlichen linsenförmiger Linsenkörper (41) in eine Form mit zwei gegeneinander geklappten Schenkeln gepresst wird,
**dadurch gekennzeichnet, dass**
die vordere Haptik (43) beim Falten des optischen Linsenkörpers (41) aus dem sich um den Linsenkörper schliessenden Raum zwischen den Halbschalen (13, 15) entweicht, indem das freie Ende (95) der Haptik in eine dafür vorgesehene Aussparung (91) in wenigstens einer der Halbschalen (13, 15) rutscht.

12. Verfahren nach dem vorangehenden Anspruch 11, **dadurch gekennzeichnet, dass** der zwischen den sich schliessenden Halbschalen entstehende Raum zylinderförmig ist, und die Aussparung (91) derart angelegt ist, dass die Haptik (43) zylindermantelseitig aus dem Raum entweichen kann.

13. Verfahren nach einem der vorangehenden Ansprüche 11-12, **dadurch gekennzeichnet, dass**
- die Linse in spannungslosem Zustand in den Hohlraum (46) eingeführt wird, und/oder
- die Linse auf der Ladefläche derart orientiert wird, dass die vordere Haptik (43) der Linse über dem Gelenk (29) derart zu liegen kommt, dass der Ansatz (93) der Haptik über der ersten Halbschale (13) und das Ende (95) der Haptik über der zweiten Halbschale (15) positioniert ist, und/oder
- der optische Linsenkörper (41) an seinen Rändern durch die beiden Halbschalen (13, 15) eingefasst ist und mit den Halbschalen (13, 15) zusammen, insbesondere in ungefähr gleicher Richtung, gefaltet wird, und/oder
- beim Schliessen der Ladekammer sich Optik und hintere Haptik absenken, während die vordere Haptik in die Aussparung (91) geführt wird.

14. Verfahren zum Falten einer intraokularen Linse und Ausstossen der Linse durch eine Injektionsdüse, beinhaltend die Schritte:
- Falten der Linse nach einem der Ansprüche 11-13, wodurch der Linsenkörper (41) ungefähr mittig gefaltet wird, sodass ein anfänglich in seinem entspannten Zustand im Wesentlichen linsenförmiger Linsenkörper in eine Form mit zwei. gegeneinander geklappten Schenkeln gepresst wird,
- Stossen des gefalteten optischen Linsenkörpers zur Injektionsdüse hin, wobei durch eine zunehmende Verengung in Richtung zur Injektionsdüse die gefaltete Linse zunehmend stärker zusammengedrückt wird,
wobei
beim Stossen zur Injektionsdüse hin die anfänglich in der Aussparung (91) positionierte vordere Haptik (43) mitgezogen wird und in einen sich mit zunehmendem Vorstossen weiter verengenden Spalt zwischen den Schenkeln des gefalteten Linsenkörpers (41) geklemmt wird.

15. Verfahren nach dem vorangehenden Anspruch 14, **dadurch gekennzeichnet, dass** die vordere Haptik (43) mitgezogen und aus der Aussparung (91) heraus über eine Kante (92) gezogen wird, wodurch die vordere Haptik (43) in einen sich mit zunehmendem Vorstossen weiter verengenden Spalt zwischen den Schenkeln des gefalteten Linsenkörpers (41) geklemmt wird.

## Claims

1. A device, in particular a loading device, for receiving and folding an intraocular lens with a lens body and at least one haptic, the device containing a first half-shell (13) and a second half-shell (15) which are connected in articulated manner to one another at a first of the longitudinal sides of the same by means of a first joint (29) and can be moved relative to one another from an open position to a closed position, the inner surfaces (17, 19) of the half-shells forming a storage surface, wherein
- in the open position, the half-shells (13, 15) form an open chamber (46) for positioning or storing the lens in the relaxed state, and
- in the closed position, the half-shells (13, 15) form an enclosed chamber (36) for positioning or storing the lens in a folded state and for ejecting the lens along the longitudinal extent of the half-shells (13, 15), **characterized in that**
a recess (91) is formed in at least one of the half-shells (13, 15), which recess is open at least from the inside of the half-shell and is suitable for receiving a front haptic (43) of the lens in the closed position of the half-shells (13, 15).

2. Device according to the preceding claim, **characterized in that**, in the closed position of the half-shells (13, 15), the recess (91) forms an area, in particular a secondary space, which is arranged longitudinally to the enclosed chamber (36) and is configured in such a way that the front haptic of the lens can be accommodated therein whereas the optics of the lens are positioned in the enclosed chamber.

3. Device according to any one of the preceding claims, **characterized in that** at least one of the half-shells (13, 15), preferably the second half-shell (15), is provided with a support (51, 103) for the front haptic (43), the support (51, 103) being suitable for guiding the front haptic, in particular the free end of the front haptic (95).

4. A device according to the preceding claim 3, **characterized in that** the support (51, 103) and the recess (91) are configured and cooperate in such a way that upon closing of the **half-shells (17, 19), from the open position to the closed position, the front haptic (43) increasingly extends beyond the support (51, 103) and out of the forming closed chamber (36) to come to rest in the recess (91) in the closed position.**

5. **Device according to any one of the preceding claims, characterized in that wings (25, 27) are respectively arranged on a second longitudinal side of the two half-shells (13, 15), so that the half-shells (13, 15) can be moved relative to one another from an open position to a closed position by means of the wings (25, 27) and by rotation about the joint (29), wherein the recess (91) preferably continues at least in one of the wings (25, 27).**

6. **Apparatus according to any one of the preceding claims, characterized in that each half-**shell (13, 15) is provided with at least one slide rail (35, 37), wherein the at least one slide rail is suitable for guiding the lens body and optionally the end of the rear haptic (44).

7. Device according to any one of the preceding claims, **characterized in that** a cover member (45) is pivotably arranged longitudinally to the first of the two half-shells (13), which cover member, in the open position, covers the open chamber (46), and in the closed position, is positioned substantially outside the enclosed chamber (39).

8. Device according to any one of the preceding claims, **characterized in that** it is designed as a cartridge for insertion into an injector, in particular into an injector housing (1).

9. Injector having an injector housing (1) with a loading device (3) provided therein, in accordance with any one of the preceding claims 1-8, a nozzle (11) mounted upstream of the loading device (3) and a plunger (9) longitudinally displaceable in the injector housing (1) toward the nozzle (11), wherein the enclosed chamber (36) can be pushed through by means of the plunger (9) for the purpose of ejecting a lens,
wherein, at least in the closed position of the half-shells (13, 15), an edge (92) is arranged on the nozzle side of the half-shells (13, 15), by means of which edge the front haptic is pressed or alternatively folded into the folded lens, in particular between the legs of the folded lens body, when the lens is pushed forward.

10. Injector according to the preceding claim 9, **characterized in that** the folding edge (92) delimits the recess (91) toward the nozzle, and, in particular, is preferably formed at a nozzle inlet (105).

11. A method of folding an intraocular lens in a device comprising the steps of:
- provision of a storage surface (17, 19) having a front region and a rear region defined by at least a first half-shell (13) and a second half-shell (15), the two half-shells (13, 15) being connected in articulated manner with one another by a first joint (29),
- placement of a lens on the storage surface (17, 19), by bringing the lens body (41) of the lens onto the storage surface (17, 19), such that, with respect to the lens body (41), a front haptic of the lens is positioned in the front region of the storage surface,
- bringing the two half-shells (13, 15) together via the first joint (29), by guiding the two half-shells (13, 15) toward one another by rotation about the first joint (29), whereby the lens body (41) is folded approximately in the center so that a lens body (41) which was initially substantially lenticular in its relaxed state is pressed into a shape with two legs folded toward one **another**
**characterized in that**
during folding of the optical lens body (41), the front haptic (43) escapes from the space between the half-shells (13, 15) that close around the lens body by the free end (95) of the haptic slipping into a recess (91) provided for this purpose in at least one of the half-shells (13, 15).

12. Method according to the preceding claim 11, **characterized in that** the space formed between the closing half-shells is cylinder-shaped, and the recess (91) is created in such a way that the haptic (43) can escape from the space on the cylinder surface side.

13. A method according to any one of the preceding claims 11-12, **characterized in that**
- the lens is inserted into the cavity (46) in an un-tensioned, and/or
- the lens is oriented on the storage surface in such a way that the front haptic (43) of the lens comes to be situated over the joint (29) such that the haptic attachment (93) is positioned over the first half-shell (13) and the end (95) of the haptic is positioned over the second half-shell (15), and/or
- the optical lens body (41) is enclosed at its edges by the two half-shells (13, 15) and is folded together with the half-shells (13, 15), in particular in approximately the same direction, and/or
- upon closing of the loading chamber, the optic and rear haptic lower whereas the front haptic is guided into the recess (91).

14. A method for folding an intraocular lens and ejecting the lens through an injection nozzle, comprising the steps of:
- folding the lens, according to any one of claims 11-13, whereby the lens body (41) is folded approximately in the center such that a lens body which was initially substantially lenticular in its relaxed state is pressed into a shape with two legs folded toward one another,
- pushing of the folded optical lens body toward the injection nozzle, wherein the folded lens is increasingly compressed by an increasing constriction in the direction toward the injection nozzle,
wherein
during the push toward the injection nozzle, the front haptic (43) that was initially positioned in the recess (91) is pulled along and is clamped in a gap between the legs of the folded lens body (41), which gap narrows further as the injection moves forward.

15. The method according to the preceding claim 14, **characterized in that** the front haptic (43) is pulled along and drawn out of the recess (91) over an edge (92), whereby the front haptic (43) is clamped into a gap between the legs of the folded lens body (41) that continues to narrow as the lens is pushed forward.

## Revendications

1. Dispositif, en particulier dispositif de chargement, pour recevoir et plier une lentille intraoculaire comprenant un corps de lentille et au moins une haptique, le dispositif contenant une première demi-coque (13) et une seconde demi-coque (15), lesquelles sont reliées entre elles de manière articulée respectivement sur une première de leurs longueurs par une première articulation (29) et peuvent être déplacées relativement entre elles d'une position ouverte à une position fermée et dont les surfaces intérieures (17, 19) forment une surface de charge, dans lequel
- les demi-coques (13, 15) forment dans la position ouverte une chambre ouverte (46) pour positionner ou stocker la lentille dans l'état détendu, et
- les demi-coques (13, 15) forment dans la position fermée une chambre (36) entourée pour positionner ou stocker la lentille dans l'état plié et pour éjecter la lentille le long de l'extension longitudinale des demi-coques (13, 15), **caractérisé en ce que**
dans au moins une des demi-coques (13, 15), un évidement (91) ouvert depuis la face intérieure de la demi-coque est formé, lequel est approprié pour recevoir une haptique avant (43) de la lentille dans la position fermée des demi-coques (13, 15).

2. Dispositif selon la revendication précédente, **caractérisé en ce que** l'évidement (91) dans la position fermée des demi-coques (13, 15) forme une zone, en particulier un espace attenant, qui est disposé sur la longueur par rapport à la chambre entourée (36) et est ainsi formé que l'haptique avant de la lentille peut être reçue dedans tandis que l'optique de la lentille est positionnée dans la chambre entourée.

3. Dispositif selon l'une des revendications précédentes, **caractérisé en ce qu'**au moins une des demi-coques (13, 15), de préférence la seconde demi-coque (15), est dotée d'un appui (51, 103) pour l'haptique avant (43), dans lequel l'appui (51, 103) est approprié pour guider l'haptique avant, en particulier l'extrémité libre de l'haptique avant (95).

4. Dispositif selon la revendication 3 précédente, **caractérisé en ce que** l'appui (51, 103) et l'évidement (91) sont formés et coopèrent de telle façon que lors de la fermeture de la position ouverte à la position fermée des demi-coques (17, 19), l'haptique avant (43) s'étend de plus en plus fortement sur l'appui (51, 103) et hors de la chambre (36) fermée en train de se former pour venir reposer dans l'évidement (91) à la position fermée.

5. Dispositif selon l'une des revendications précédentes, **caractérisé en ce que** des ailettes (25, 27) sont disposées sur respectivement une seconde longueur des deux demi-coques (13, 15), de façon à ce que les demi-coques (13, 15), au moyen des ailettes (25, 27) et par rotation sur l'articulation (29), puissent être déplacées l'une par rapport à l'autre d'une position ouverte à une position fermée, dans lequel de préférence, l'évidement (91) se poursuit au moins dans une des ailettes (25, 27).

6. Dispositif selon l'une des revendications précédentes, **caractérisé en ce que** chaque demi-coque (13, 15) est dotée d'au moins un rail de coulissement (35, 37), dans lequel l'au moins un rail de coulissement est approprié pour guider le corps de lentille et en option l'extrémité de l'haptique arrière (44).

7. Dispositif selon l'une des revendications précédentes, **caractérisé en ce qu'**un membre de couvercle (45) est disposé pivotant sur la longueur sur la première des deux demi-coques (13), lequel couvre la chambre ouverte (46) dans la position ouverte et est positionné essentiellement à l'extérieur de la chambre entourée (39) dans la position fermée.

8. Dispositif selon l'une des revendications précédentes, **caractérisé en ce que** celui-ci est formé en tant que cartouche pour l'insertion dans un injecteur, en particulier dans un logement d'injecteur (1).

9. Injecteur comprenant un logement d'injecteur (1) avec un dispositif de chargement (3) prévu à l'intérieur selon l'une des revendications précédentes 1-8, une buse (11) disposée à l'avant du dispositif de charge (3) et un poussoir (9) pouvant être déplacé en longueur en direction de la buse (11) dans le logement d'injecteur (1), dans lequel la chambre entourée (36) peut être traversée au moyen du poussoir (9) dans le but d'éjecter une lentille,
dans lequel au moins dans la position fermée des demi-coques (13, 15), une arête (92) est disposée côté buse des demi-coques (13, 15), à travers laquelle, lors de l'avancée de la lentille, l'haptique avant est enfoncée, respectivement pliée dans la lentille pliée, en particulier entre les branches du corps de lentille plié.

10. Injecteur selon la revendication précédente 9, **caractérisé en ce que** l'arête de pli (92) délimite l'évidement (91) en direction de la buse, est formée en particulier de préférence sur une entrée de buse (105).

11. Procédé de pliage d'une lentille intraoculaire dans un dispositif, comprenant les étapes de :
- fourniture d'une surface de charge (17, 19) avec une partie avant et une partie arrière, laquelle est définie au moins par une première demi-coque (13) et une seconde demi-coque (15), dans lequel les deux demi-coques (13, 15) sont reliées entre elles de manière articulée par une première articulation (29),
- d'amenée d'une lentille sur la surface de charge (17, 19), en ce que le corps de lentille (41) de la lentille est amené sur la surface de charge (17, 19), de façon à ce que par rapport au corps de lentille (41), une haptique avant de la lentille soit positionnée dans la partie avant de la surface de charge,
- de réunion des deux demi-coques (13, 15) sur la première articulation (29), en ce que les deux demi-coques (13, 15) sont amenées l'une sur l'autre par rotation sur la première articulation (29), ce par quoi le corps de lentille (41) est plié environ en son milieu, de sorte qu'un corps de lentille (41) essentiellement en forme de lentille au départ dans son état détendu est pressé en une forme avec deux branches rabattues l'une contre l'autre,
**caractérisé en ce que**
l'haptique avant (43), lors du pliage du corps de lentille (41) optique, s'évacue hors de l'espace se refermant autour du corps de lentille entre les demi-coques (13, 15) **en ce que** l'extrémité libre (95) de l'haptique glisse dans un évidement (91) prévu à cet effet dans au moins une des demi-coques (13, 15).

12. Procédé selon la revendication précédente 11, **caractérisé en ce que** l'espace produit entre les demi-coques se refermant est cylindrique, et l'évidement (91) est ainsi conçu que l'haptique (43) peut s'évacuer de l'espace côté enveloppe du cylindre.

13. Procédé selon l'une des revendications précédentes 11-12, **caractérisé en ce que**
- la lentille est introduite dans l'espace creux (46) dans l'état sans tension, et/ou
- la lentille est ainsi orientée sur la surface de chargement que l'haptique avant (43) de la lentille vient reposer sur l'articulation (29) de telle façon que l'épaulement (93) de l'haptique est positionné sur la première demi-coque (13) et l'extrémité (95) de l'haptique est positionnée sur la seconde demi-coque (15), et/ou
- le corps de lentille optique (41) est saisi sur ses bords par les deux demi-coques (13, 15) et est plié conjointement avec les demi-coques (13, 15) en particulier dans environ la même direction, et/ou
- lors de la fermeture de la chambre de chargement, l'optique et l'haptique arrière s'abaissent tandis que l'haptique avant est dirigée dans l'évidement (91).

14. Procédé de pliage d'une lentille intraoculaire et d'éjection de la lentille au travers d'une buse d'injection, comprenant les étapes de :
- pliage de la lentille selon l'une des revendications 11-13, ce par quoi le corps de lentille (41) est plié environ en son milieu, de sorte qu'un corps de lentille essentiellement en forme de lentille au départ dans son état détendu est pressé en une forme avec deux branches rabattues l'une contre l'autre,
- poussée du corps de lentille optique plié en direction de la buse d'injection, dans lequel par un rétrécissement croissant en direction de la buse d'injection, la lentille pliée est comprimée de plus en plus fort,
dans lequel
lors de la poussée vers la buse d'injection, l'haptique avant (43) positionnée au départ dans l'évidement (91) est entraînée et se bloque dans une fente entre les branches du corps de lentille (41) plié qui se réduit de plus en plus à mesure que la poussée vers l'avant augmente.

15. Procédé selon la revendication 14, **caractérisé en ce que** l'haptique avant (43) est entraînée et tirée hors de l'évidement (91) sur une arête (92), ce par quoi l'haptique avant (43) se bloque dans une fente entre les branches du corps de lentille (41) plié qui se réduit de plus en plus à mesure que la poussée vers l'avant augmente.
